# EUROPÄISCHE PATENTSCHRIFT

(11) **EP 3 902 578 B1**
(45) Veröffentlichungstag und Bekanntmachung des Hinweises auf die Patenterteilung: **02.10.2024**
(21) Anmeldenummer: 19836500.9
(22) Anmeldetag: 27.12.2019
(51) Int. Cl.: A61M 1/16, C02F 1/00

(54) **DIALYSEMASCHINE ENTHALTEND EINE VORRICHTUNG ZUR AUFBEREITUNG VON FLÜSSIGKEITEN, INSBESONDERE WASSER, UND VERFAHREN ZUR ÜBERWACHUNG DER VORRICHTUNG AUF KONTAMINATION**
DIALYSIS MACHINE COMPRISING A SYSTEM FOR PREPARATION OF FLUIDS, WATER IN PARTICULAR, AND METHODS FOR MONITORING CONTAMINATION OF THE SYSTEM
MACHINE DE DIALYSE COMPRENANT UN SYSTEME DE PREPARATION DE FLUIDES, DE L'EAU EN PARTICULIER, ET METHODES DE SURVEILLANCE DE CONTAMINATION DU SYSTEME

(30) Priorität: 28.12.2018 DE 102018133664
(43) Veröffentlichungstag der Anmeldung: 03.11.2021
(73) Patentinhaber: Fresenius Medical Care Deutschland GmbH, 61352 Bad Homburg (DE)
(72) Erfinder: SMYSLOVA, Liubov, 61352 Bad Homburg (DE); WIESEN, Gerhard, 61352 Bad Homburg (DE)
(74) Vertreter: Ricker, Mathias
(86) Internationale Anmeldenummer: PCT/EP2019/087057
(87) Internationale Veröffentlichungsnummer: WO 2020/136230

(56) Entgegenhaltungen:
- WO-A1-01/02035
- DE-C1- 4 118 625
- US-A- 5 660 722
- US-A1- 2002 158 019
- US-B1- 6 655 394

## Beschreibung

### THEMA DER ERFINDUNG

Die Erfindung betrifft eine Dialysemaschine, enthaltend eine Vorrichtung zum Aufbereiten von Flüssigkeiten, insbesondere der Aufbereitung von Wasser für die Dialyse, an einem oder mehreren Hohlfasermembranfiltern, sowie die entsprechend beschriebenen Ausgestaltungen der Vorrichtung an sich. Die Erfindung betrifft weiterhin ein Verfahren zur Überwachung der Vorrichtung zur Aufbereitung von Flüssigkeiten, insbesondere Wasser, auf Kontamination.

### HINTERGRUND DER ERFINDUNG

Im Bereich der Hämodialyse werden große Mengen Wasser für die Herstellung von Dialyseflüssigkeiten benötigt. Während einer üblichen Dialysetherapie eines nierenkranken Menschen werden bis zu 120 Liter an Dialysierflüssigkeit verbraucht. Die für die Therapie bereitgestellte Dialysierflüssigkeit muss für die Therapie ein hohes Maß an Reinheit und Kontaminationsfreiheit aufweisen, um eine pharmazeutisch annehmbare therapeutische Behandlung der nierenkranken Patienten zu ermöglichen. Bekannt sind Dialysemaschinen, die einen Hydraulikkreislauf aufweisen, der im Wesentlichen die Herstellung, Förderung und Bereitstellung von Dialyseflüssigkeit gewährleistet. In bestimmten Anwendungen wird dabei die Dialyseflüssigkeit in der Dialysemaschine aus bereitgestellten Dialysekonzentraten und aufbereitetem Wasser hergestellt. Das Wasser wird dabei in einer Mischkammer mit den Dialysekonzentraten vermischt und zur gebrauchsfertigen Dialyseflüssigkeit umgesetzt.

Aus Gründen der Hygiene wird für die Herstellung von Dialyseflüssigkeiten Wasser mit einem möglichst hohen Reinheitsgrad verwendet. Für die Herstellung von Dialyseflüssigkeit wird vorzugsweise Reinstwasser verwendet, das eine elektrische Leitfähigkeit von 10⁻⁴ S/m und weniger aufweist. Trotz diesem hohen Reinheitsgrad ist es weiter notwendig, das bereitgestellte Reinstwasser durch einfache oder mehrfache Filtration aufzubereiten und von Kontaminanten zu befreien, die in der Therapie schädliche Auswirkungen für den Patienten haben können. Insbesondere sind hier auch solche partikulären Kontaminanten zu nennen, die nicht zur Leitfähigkeitserhöhung des Wassers beitragen. Solche Kontaminanten können beispielsweise Bakterien, Viren, Endotoxine oder Pilze sein.

Bekannt sind daher Dialysemaschinen und Wasseraufbereitungsanlagen für die Dialyse, die Wasseraufbereitungseinheiten aufweisen, in denen bereitgestellte Flüssigkeit, insbesondere Wasser, mit einem geringeren Reinheitsgrad durch einfache oder mehrfache Filtration für die Dialyse bereitgestellt wird, wie in der DE 36 41 843 A1 beschrieben. Die so aufbereitete Flüssigkeit, insbesondere Wasser, kann unter Verwendung von weiteren Bestandteilen, z.B. Dialysekonzentraten, zu Dialyseflüssigkeiten mit dem erforderlichen Reinheitsgrad weiterverarbeitet werden. Für die Herstellung der Dialysierflüssigkeiten kann dabei von reinem Wasser ausgegangen werden, das aufbereitet wird und mit weiteren Bestanteilen einer Dialysierflüssigkeit zur gebrauchsfertigen Dialysierflüssigkeit umgesetzt wird. Es kann aber auch von einer wässrigen Vorläuferflüssigkeit ausgegangen werden, die bereits Bestandteile der gebrauchsfertigen Dialysierflüssigkeit enthält, z.B. Elektrolyte, die weiter aufbereitet wird und mit weiteren Bestandteilen einer Dialysierflüssigkeit zur gebrauchsfertigen Dialysierflüssigkeit umgesetzt wird.

Während der Aufbereitung des Wassers für die Verwendung zur Herstellung von Dialysierflüssigkeit, wird eine zur Verfügung stehende Flüssigkeit, insbesondere Wasser, durch einen Filter filtriert. Üblicherweise werden dabei Hohlfasermembranfilter verwendet, deren Membraneigenschaften so beschaffen sind, dass Kontaminanten, die in der Flüssigkeit, insbesondere Wasser vorliegen, von der Membranwandung der Hohlfasermembran bei der Filtration zurückgehalten werden können. Die Hohlfasermembranen derartiger Hohlfasermembranfilter weisen dabei ein Ausschlussgrenze auf, die Kontaminanten mit einer Größe von beispielsweise 10 nm oder 20 nm zurückhalten können. Weil nach Möglichkeit alle Kontaminanten durch die Filtration zurückgehalten werden, werden die Kontaminanten in den Hohlfasermembranfiltern im Laufe einer andauernden Aufbereitung von Flüssigkeit, insbesondere Wasser für die Dialyse, angereichert. Eine zunehmende Anreicherung der Kontaminanten in den Filtern ist allerding bei der Wasseraufbereitung unerwünscht. Weiterhin werden üblicherweise in der Dialyse auch sogenannte Substitutionsflüssigkeiten aus bereitgestellten Flüssigkeiten, insbesondere Wasser, durch Wasseraufbereitung und Filtration bereitgestellt. Solche Substitutionsflüssigkeiten werden dem Dialysepatienten in das extrakorporal geführte Blut des extrakorporalen Blutkreislaufs verabreicht. Kontaminationen dieser Substitutionsflüssigkeiten durch Kontaminanten, die durch schadhafte Hohlfasermembranfilter der Wasseraufbereitungsanlagen verursacht werden, greifen damit direkt in den Organismus des Patienten ein und sind daher für den Patienten äußerst gefährlich und in jedem Fall zu vermeiden

Die US 2009/217777 verweist auf das Problem der Detektion von Kontaminanten, die in Produktionsprozessen, z.B. in Trinkwasser oder in Stoffen medizinischer Verwendung vorliegen können. Dazu beschreibt die US 2009/0217777 Verfahren und eine Vorrichtung zum Konzentrieren eines Analyten, der sich in einer Flüssigkeit befindet. Die Verfahren umfassen die Schritte des Bildens und Sammelns eines Retentats, das einen Analyten enthält. Das Retentat wird gebildet, indem eine Flüssigkeit, die einen Analyten enthält, durch eine Ultrafiltrationsmembran geleitet wird, die der Analyt aufgrund des Größenausschlusses nicht passieren kann. Die Ultrafiltrationsmembran trennt einen Bereich einer Retentatseite, auf der sich der Analyt anreichert von einem Bereich einer Permeatseite, auf der die filtrierte Flüssigkeit anfällt. Das Retentat wird gesammelt, indem das Filtrat mit einem Gas von der Permeatseite der Ultrafiltermembran verdrängt wird, die Retentatseite der Ultrafiltermembran mit Gas und mit Flüssigkeit gespült wird, um eine Retentatlösung herzustellen, die das Retentat und den Analyten enthält, und die Retentatlösung aufgefangen wird.

Angesichts der besonderen Bedingungen, die für die Aufbereitung von Flüssigkeiten oder Wasser für die Dialyse einzuhalten sind, erweisen sich die im Stand der Technik bekannten Methoden und Vorrichtungen zur Überwachung und Detektion von Kontaminanten in einer Dialysemaschine als unzureichend. Es besteht daher in der Aufbereitung von Flüssigkeiten, insbesondere in der Aufbereitung von Wasser für die Dialyse, ein andauerndes Bedürfnis die Wasseraufbereitung innerhalb einer Dialysemaschine aus medizinischer Sicht sicherer für den Patienten zu gestalten. Insbesondere besteht daher das Bedürfnis, den Prozess der Aufbereitung von Flüssigkeiten, insbesondere von Wasser für die Dialyse, überwachen zu können und eine Methode bereitzustellen, mit der die Qualität der aufbereiteten Flüssigkeit, insbesondere des Wassers, sowie die Kontamination der Aufbereitungsanlage innerhalb einer Dialysemaschine zuverlässig während der Aufbereitung getestet werden kann, so dass bei unzulässig hoher Kontamination einer Vorrichtung zur Aufbereitung einer Flüssigkeit, insbesondere Wasser, entsprechende Rückschlüsse gewonnen werden können. Gegebenenfalls darauf können darauffolgend entsprechende Maßnahmen eingeleitet werden, wonach eine mögliche Kontamination der aufzubereitenden Flüssigkeit, insbesondere Wasser, vermieden werden kann, indem z.B. betroffene Funktionsteile der Aufbereitungsanlage ausgetauscht werden können.

### AUFGABE DER ERFINDUNG

In einem ersten Aspekt der Erfindung bestand daher die Aufgabe, eine Vorrichtung bzw. eine Dialysemaschine, enthaltend eine Vorrichtung zur Aufbereitung von Flüssigkeiten, insbesondere eine Vorrichtung zur Aufbereitung von Wasser bereitzustellen, die eine Überwachung von Kontaminanten während der Aufbereitung der Flüssigkeit, insbesondere des Wassers, ermöglicht.

In einem weiteren Aspekt der Erfindung bestand die Aufgabe ein Verfahren zur Überwachung der Aufbereitung von Flüssigkeiten, insbesondere der Aufbereitung von Wasser für die Dialyse, bereitzustellen, mit dem die Kontamination der Vorrichtung bzw. der Dialysemaschine, enthaltend eine Vorrichtung zur Aufbereitung von Flüssigkeiten, insbesondere der Vorrichtung zur Aufbereitung von Wasser für die Dialyse, festgestellt werden kann.

### ZUSAMMENFASSUNG DER ERFINDUNG

Erfindungsgemäß wird in einem ersten Aspekt der Erfindung die Aufgabe durch eine Vorrichtung zur Aufbereitung von Flüssigkeiten, insbesondere Wasser, nach Anspruch 1 gelöst. Die Unteransprüche 2 bis 7 stellen bevorzugte Ausführungsformen da. Patentanspruch 15 betrifft ferner eine Dialysemaschine, enthaltend eine Vorrichtung gemäß den Ansprüchen 1 bis 7.

Erfindungsgemäß wird in einem weiteren Aspekt die Aufgabe durch ein Verfahren zur Überwachung der Aufbereitung von Flüssigkeiten, insbesondere Wasser nach Anspruch 8 gelöst. Die Ansprüche 8 bis 14 stellen weitere Ausführungsformen da.

In der nachfolgenden detaillierten Beschreibung werden die erfindungsgemäße Dialysemaschine und die erfindungsgemäße Vorrichtung stets gemeinsam beschrieben. Die dort erwähnten bevorzugten Ausführungsformen beziehen sich auf die Vorrichtung an sich, sowie die Dialysemaschine enthaltend die jeweilige Vorrichtung.

### DETAILLIERTE BESCHREIBUNG DER ERFINDUNG

Ein erster Aspekt der Erfindung betrifft eine Vorrichtung bzw. eine Dialysemaschine, enthaltend eine Vorrichtung zur Aufbereitung einer Flüssigkeit, insbesondere zur Aufbereitung von Wasser für die Dialyse,
aufweisend einen ersten Hohlfasermembranfilter, wobei der erste Hohlfasermembranfilter weiter aufweist,
eine Vielzahl von Hohlfasermembranen, die einen Retentatraum und einem Filtratraum bilden, wobei der Retentatraum und der Filtratraum über die semipermeable Membranwandungen der Hohlfasermembranen voneinander getrennt sind,
einen Fluidanschluss zur Zuführung von Flüssigkeit, insbesondere Wasser in den Retentatraum,
einen Fluidanschluss zur Ableitung von Flüssigkeit, insbesondere Wasser, aus dem Retentatraum,
wenigstens einen Fluidanschluss zur Ableitung von Flüssigkeit, insbesondere Wasser, aus dem Filtratraum,
wobei die Vorrichtung weiter aufweist,
eine Fluidleitung in Flüssigkeitsverbindung mit dem Fluidanschluss zur Zuführung von Flüssigkeit, insbesondere Wasser, in den Retentatraum des ersten Hohlfasermembranfilters,
eine Fluidleitung in Flüssigkeitsverbindung mit dem Fluidanschluss zur Ableitung von Flüssigkeit, insbesondere Wasser, aus dem Retentatraum des ersten Hohlfasermembranfilters,
eine Fluidleitung in Flüssigkeitsverbindung mit dem Fluidanschluss zur Ableitung von Flüssigkeit, insbesondere Wasser, aus dem Filtratraum des ersten Hohlfasermembranfilters,
eine erste Ventilschaltung im Eingriff mit der Fluidleitung zum Ableiten von Flüssigkeit, insbesondere Wasser, aus dem Retentatraum, wobei die Ventilschaltung wenigstens zwei Ventilstellungen umfasst, wonach der Durchfluss von Flüssigkeit durch die Fluidleitung zum Ableiten von Flüssigkeit aus dem Retentatraum, blockiert werden kann oder durchgeleitet werden kann,
optional weiter aufweisend
eine erste Fluidverzweigung in der Fluidleitung zur Ableitung von Flüssigkeit, insbesondere Wasser, aus dem Retentatraum des ersten Hohlfasermembranfilters, in Flüssigkeitsverbindung mit einer ersten abzweigende Fluidleitung, eine zweite Ventilschaltung im Eingriff mit der ersten abzweigenden Fluidleitung, wobei die zweite Ventilschaltung wenigstens zwei Ventilstellungen umfasst, wonach der Durchfluss von Flüssigkeit, insbesondere Wasser durch die erste abzweigende Fluidleitung blockiert werden kann oder durchgeleitet werden kann,
dadurch gekennzeichnet, dass
stromabwärts der ersten Ventilschaltung in Flüssigkeitsverbindung mit der Fluidleitung zum Ableiten von Flüssigkeit, insbesondere Wasser, aus dem Retentatraum, eine Probeentnahmestelle angeordnet ist
und/ oder, dass
stromab der zweiten Ventilschaltung in Flüssigkeitsverbindung zu der ersten abzweigenden Fluidleitung eine zweite Probenentnahmestelle angeordnet ist.
Die vorliegend definierte Vorrichtung ist als Teil einer Wasseraufbereitungseinheit in einer Dialysemaschine einsetzbar.

Die Vorrichtung bzw. Dialysemaschine, aufweisend eine Vorrichtung nach dem ersten Aspekt der Erfindung weist den Vorteil auf, dass während eines Verfahrens zur Aufbereitung von Flüssigkeit, insbesondere Wasser für die Dialyse, in einer Dialysemaschine durch Einstellen der einen ersten Ventilschaltung Proben entnommen werden können und der Analyse auf Kontamination zugeführt werden können. Insbesondere wird durch Einstellen der einen ersten Ventilschaltung in eine blockierende Stellung Flüssigkeit, insbesondere Wasser, die in den Retentatraum des einen ersten Hohlfasermembranfilters eingeleitet wird und einer "dead-end" Filtration unterzogen. Die eingeleitete Flüssigkeit, insbesondere Wasser, tritt über die Membranwand in den Filtratraum über und wird dabei filtriert. Die Hohlfasermembranen des einen ersten Hohlfasermembranfilters sind dabei so beschaffen, dass Kontaminanten von der Membranwandung der Hohlfasermembranen während der "dead-end" Filtration zurückgehalten werden und sich im Retentatraum anreichern.

Das Einleiten der Flüssigkeit, insbesondere Wasser, in den Retentatraum des ersten Hohlfasermembranfilters und das Überleiten der Flüssigkeit, insbesondere Wasser, über die Membranwandung der Hohlfasermembranen in den Filtratraum kann über geeignete Pumpenmittel erfolgen, wie z.B. durch Schlauchrollenpumpen, Zahnradpumpen oder Impellerpumpen. Dem Fachmann sind geeignete und übliche Pumpenmittel im Bereich der Dialysetechnik bekannt.

Die Anreicherung der Kontaminanten im Retentatraum des ersten Hohlfasermembranfilters kann überprüft werden, indem die Ventilstellung der ersten Ventilschaltung so eingestellt wird, dass durch die Fluidleitung zum Ableiten von Flüssigkeit aus dem Retentatraum, Flüssigkeit abgeleitet werden kann. In dieser Einstellung der Ventilschaltung werden die angereicherten Kontaminanten aus dem Retentatraum freigespült und können stromabwärts der ersten Ventilschaltung, über die eine erste Probeentnahmestelle entnommen werden und die Proben einer Analyse zugeführt werden. Hierbei verbleibt die gegebenenfalls vorhandene Ventilstellung der zweiten Ventilschaltung in eine blockierende Stellung.

Alternativ kann die Anreicherung der Kontaminanten im Retentatraum des ersten Hohlfasermembranfilters auch überprüft werden, indem die Ventilstellung der ersten Ventilschaltung in einer blockierenden Stellung verbleibt und die Ventilstellung der zweiten Ventilschaltung in eine durchleitende Ventilstellung gebracht wird. In dieser Einstellung der Ventilschaltungen werden die angereicherten Kontaminanten aus dem Retentatraum freigespült und können, über die zweite Probeentnahmestelle entnommen werden und die Proben einer Analyse zugeführt werden.

Vorteilhafterweise kann wiederholt nach vorherbestimmten Mengen an Flüssigkeit, insbesondere Wasser, die in den Filtratraum über die Membranwandungen der Hohlfasermembranen überführt wurde, eine Probe an der ersten oder zweiten Probeentnahmestelle entnommen werden und auf mögliche Kontamination untersucht werden und Rückschlüsse auf den Kontaminationsgrad der Anlage oder auf die bereitgestellte Flüssigkeit, insbesondere Wasser, geschlossen werden. Sofern in der bereitgestellten Flüssigkeit eine geringe Kontamination zu erwarten ist, kann die Anreicherung der Kontaminanten im Retentatraum über einen langen Zeitraum fortgeführt werden, bis eine Anreicherung der Kontaminanten über eine analytisch möglich Nachweisgrenze steigt und damit sicher eine Kontamination nachgewiesen werden kann, die ohne eine solche Anreicherung unentdeckt bliebe. Dies ist aus hygienischer Sicht eine Dialysetherapie wichtig, da eine schädigende Wirkung von Kontaminanten für den Patienten schon unterhalb einer nachweisbaren Menge an Kontaminanten einsetzen kann. Die Anreicherung von Kontaminanten lässt damit den Rückschluss zu, ob die zur Verfügung stehende Flüssigkeit, insbesondere Wasser, nicht schon einen unzulässigen hohen Kontaminationsgrad aufweist. In der Praxis kann die vorherbestimmte Menge, die wie oben beschrieben als Filtrat über den Filtratraum des ersten Hohlfasermembranfilters gewonnen wird, je nach Kontaminationsgrad der zur Verfügung stehenden Flüssigkeit, insbesondere Wasser, 20 Liter oder mehr, 30 Liter oder mehr, 50 Liter, oder mehr 100 Liter oder mehr, oder 200 Liter oder mehr betragen.

Für die Vorrichtung zur Aufbereitung einer Flüssigkeit wird ein Hohlfasermembranfilter mit Hohlfasermembranen verwendet, deren Membraneigenschaft dadurch ausgezeichnet ist, dass die Kontaminanten zurückgehalten werden können. Die Porengröße der selektiven Schicht der Hohlfasermembran steht dabei mit der Zurückhaltung der Kontaminanten im Zusammenhang. Unter einer "*Hohlfasermembran"* ist in diesem Zusammenhang eine hohle Faser zu verstehen deren Wandung porös ist und für Flüssigkeiten, insbesondere Wasser, durchdringbar ist. Sofern durch die Aufbereitung der Flüssigkeit z.B. auch Viren zurückgehalten werden sollen, ist die Verwendung von Hohlfasermembranen mit einer maximalen mittlere Porengröße kleiner 20 nm, bevorzugt kleiner 10 nm, ratsam.

Erfindungsgemäß verwendbare Hohlfasermembranfilter und die baulichen Einzelheiten solcher Hohlfasermembranfilter sind dem Fachmann aus dem Stand der Technik hinlänglich bekannt. Es wird in diesem Zusammenhang auf die DE3641843A1 verwiesen.

Bei dem erfindungsgemäß verwendeten Hohlfasermembranfilter ist der Innenraum des Filtermoduls in einen "*Retentraum"* und einen *"Filtratraum"* unterteilt, die durch die Membranwandung der Hohlfasermembranen und durch die endseitigen Harzvergusszonen, voneinander getrennt. Der "*Retentatraum"* des Filtermoduls ist für die Rückhaltung der Kontaminanten vorgesehen. Die aufzubereitende Flüssigkeit kann durch Filtration über die Membranwand hinweg in den "*Filtratraum"* des Hohlfasermembranfilters gelangen. Nicht filtrierte Flüssigkeit und Kontaminanten verbleiben im Retentatraum und werden als *"Retentat"* bezeichnet. Die durch den Übergang über die Membranwand in den Filtratraum gelangte Flüssigkeit wird als *"Filtrat"* bezeichnet.

Unter einer "Dead-End" Filtration ist im Sinne der vorliegenden Erfindung ein Verfahren zu verstehen bei der Flüssigkeit, insbesondere Wasser, in den Retentatraum des Hohlfasermembranfilters über einen Fluidanschluss eingeleitet wird, gleichzeitig aber ein Ableiten der Flüssigkeit aus dem Retentatraum über die blockierende Stellung des ersten Ventils gesperrt ist. Die in den Retentatraum einströmende Flüssigkeit, insbesondere Wasser, wird dann über einen sich aufbauenden Druckgradienten über die Membranwand in den Filtratraum geleitet. In der Regel wird das "Dead-End"-Verfahren an Hohlfasermembranfiltern durchgeführt, indem Flüssigkeit über einen Flüssigkeitszugang in den Hohlfasermembranfilter auf die Lumenseite der Hohlfasermembranen eingeleitet wird. Die Enden der Hohlfasermembranen sind dabei verschlossen oder blockiert, so dass keine Flüssigkeit über die Enden der Hohlfasermembranen austreten kann. Insbesondere wird häufig der zweite Flüssigkeitszugang am anderen Ende des Hohlfasermembranfilters blockiert, so dass sämtliche zu filtrierende Flüssigkeit die Membranwand passiert und als Filtrat abgeleitet werden kann.

Im Sinne der vorliegenden Anmeldung wird unter einem "*Fluidanschluss"* ein Anschluss am Filtermodul verstanden, über den Flüssigkeit, insbesondere Wasser für die Dialyse, in den Hohlfasermembranfilter, z.B. entweder in den Retentatraum oder den Filtratraum, eingeleitet werden kann. Ein derart bezeichneter Fluidzugang eignet sich gleichermaßen auch zum Ableiten von Flüssigkeit, insbesondere zum Ableiten von Filtrat oder Retentat aus dem Retentatraum oder Filtratraum eines Filtermoduls.

Die bereitgestellte Flüssigkeit, insbesondere das bereitgestellte Wasser, werden über Pumpenmittel in den Retentatraum gefördert. Als "*Pumpenmittel"* wird im Sinne der vorliegenden Anmeldung jedes Mittel verstanden, dass durch eine Druckveränderung Flüssigkeiten zu fördern vermag. Für das erfindungsgemäße Verfahren ist insbesondere die Verwendung von Membranpumpen oder peristaltischen Pumpen, insbesondere Schlauchrollenpumpen, wie sie im Bereich der Medizintechnik hinlänglich bekannt sind, vorgesehen. Es ist generell im Sinne der vorliegenden Anmeldung zu verstehen, dass geeignete Pumpenmittel das Zuführen und Ableiten von Flüssigkeiten in durch die Fluidleitungen, Ventilschaltungen, Fluidanschlüsse und Hohlfasermembranfilter unterstützen, wo dies für die Aufbereitung der Flüssigkeit, insbesondere Wasser, notwendig ist.

Als "*Fluidleitungen"* kann jedwedes Verbindungssystem dienen, das zur Förderung der in der erfindungsgemäßen Vorrichtung vorgesehenen Flüssigkeiten, insbesondere Wasser, geeignet ist. Insbesondere können flexible Schlauchsysteme oder Rohrsysteme als Fluidleitungen dienen, wie sie in der Medizintechnik, insbesondere in der Dialyse, bekannt sind.

Unter "*Fluidverzweigung"* wird im Sinne der vorliegenden Anmeldung das Zusammenlaufen mindestens dreier Fluidleitungen in einer baulichen Einheit verstanden. In der Dialysetechnik sind dem Fachmann entsprechende Bauteile bekannt, wie z.B. sogenannte "T-Stücke" oder "Y-Stücke". Entsprechend ist unter einer Fluidverzweigung zu verstehen, dass Flüssigkeit, die durch eine Fluidleitung geleitet wird in mindestens zwei weiteren Fluidleitungen weitergeleitet wird. Als Fluidverzweigung wird im Sinne der vorliegenden Anmeldung auch verstanden, dass Flüssigkeit, die durch mindestens zwei Fluidleitungen geleitet wird, in einer weiteren Fluidleitung weitergeleitet wird.

Unter den Begriffen "*stromaufwärts"* und "*stromabwärts"* wird in der vorliegenden Anmeldung eine Wegrichtung der Flüssigkeit, insbesondere Wasser, verstanden, die durch die erfindungsgemäße Vorrichtung geleitet wird. Im Verständnis der vorliegenden Anmeldung ist die "*Wegrichtung"* an der Basisströmungsrichtung der Flüssigkeit, insbesondere Wasser, in der erfindungsgemäßen Vorrichtung im Sinne einer Aufbereitung und gegebenenfalls mehrfachen Filtration der bereitgestellten Flüssigkeit, insbesondere Wasser ausgerichtet. Als "*Basisströmungsrichtung"* wird vorliegend verstanden:
- das Leiten von Flüssigkeit, insbesondere Wasser, durch Fluidleitung und Fluidanschluss in den Retentatraum eines Hohlfasermembranfilters,
- gegebenenfalls das Ableiten der Flüssigkeit, insbesondere Wasser aus dem Retentatraum des Hohlfasermembranfilters,
- das Überleiten von Flüssigkeit, insbesondere Wasser, über die Membranwandung in den Filtratraum des Hohlfasermembranfilters
- das Ableiten von Flüssigkeit, insbesondere Wasser, aus dem Filtratraum eines Hohlfasermembranfilters durch Fluidanschluss und Fluidleitung.
- Das Leiten von Flüssigkeit, insbesondere Wasser, zu Entnahmestellen und Probenentnahmestellen gegebenenfalls durch abzweigende Fluidleitungen.
Eine Wegrichtung entlang dieser erläuterten Strömungsrichtung wird als *"stromaufwärts"* bezeichnet. Eine Wegrichtung entgegen dieser erläuterten Strömungsrichtung wird als *"stromabwärts"* bezeichnet. In der vorliegenden Anmeldung werden auch Strömungsrichtungen beschrieben, die entgegen der oben beschriebenen Strömungsrichtung verlaufen. Die Begriffe *"stromaufwärts"* und *"stromabwärts"* sind gemäß der vorliegenden Anmeldung nicht auf diese entgegengesetzten Strömungsrichtungen anwendbar.

Unter dem Begriff *"Aufbereitung* einer Flüssigkeit", insbesondere "Aufbereitung von Wasser" wird gemäß der vorliegenden Anmeldung ein Verfahren verstanden in dem Verunreinigungen, insbesondere Kontaminanten, in einer bereitgestellten Flüssigkeit, insbesondere Wasser, abgereichert werden, um die aufbereitete Flüssigkeit oder, insbesondere das aufbereitete Wasser, einer weiteren Verwendung zugänglich zu machen. Unter einer *"Abreicherung"* ist eine Verringerung der Konzentration von Verunreinigungen oder Kontaminanten zu verstehen. Am Beispiel der Aufbereitung von Wasser für die Dialyse kann dies bedeuten, dass Trinkwasser, dass z.B. durch das Trinkwasserversorgungssystem der Städte oder Gemeinden zur Verfügung gestellt wird einer weiteren Reinigung unterzogen wird, um es der Verwendung für die therapeutische Behandlung von Dialysepatienten zugänglich zu machen. Die erforderliche Wasserqualität für die Dialyse wird in der Norm ISO13359:2014, die erforderliche Qualität für Dialysierflüssigkeit wird in der Norm ISO11663:2014 beschrieben. Insbesondere kann Wasser auch über einen lonentauscher oder eine Umkehrosmoseanlage zur Verfügung gestellt werden.

In einer Ausführungsform gemäß dem ersten Aspekt der Erfindung ist die erfindungsgemäße Vorrichtung bzw. Dialysemaschine, enthaltend eine Vorrichtung dadurch gekennzeichnet, dass von der Fluidleitung zur Zuführung von Flüssigkeit, insbesondere Wasser, in den Retentatraum stromaufwärts des ersten Hohlfasermembranfilters eine zweite Fluidabzweigung angeordnet ist, weiter gekennzeichnet dadurch, dass in der Fluidleitung zur Ableitung von Flüssigkeit, insbesondere Wasser, aus dem Filtratraum des ersten Hohlfasermembranfilters eine dritte Fluidabzweigung angeordnet ist,
weiter gekennzeichnet dadurch, dass die zweite Fluidabzweigung an der Fluidleitung zur Zuführung von Flüssigkeit, insbesondere Wasser, in den Retentatraum (F_{1F}) des ersten Hohlfasermembranfilters und die dritte Fluidabzweigung an der Fluidleitung zur Ableitung von Flüssigkeit, insbesondere Wasser aus dem Filtratraum des ersten Hohlfasermembranfilters, über eine Fluidleitung flüssigkeitsverbindend verbunden sind, weiter gekennzeichnet dadurch, dass im Eingriff mit der Fluidleitung zwischen der zweiten Fluidabzweigung und der dritten Fluidabzweigung eine dritte Ventilschaltung angeordnet ist, wobei die dritte Ventilschaltung wenigstens zwei Ventilstellungen umfasst, wonach der der Durchfluss von Flüssigkeit, insbesondere Wasser, durch die Fluidleitung zwischen der zweiten Fluidabzweigung und der dritten Fluidabzweigung blockiert werden kann oder durchgeleitet werden kann,
weiter dadurch gekennzeichnet, dass stromabwärts der dritten Fluidabzweigung im Eingriff mit der Fluidleitung zur Ableitung von Flüssigkeit, insbesondere Wasser, aus dem Filtratraum eine vierte Ventilschaltung angeordnet ist, wobei die vierte Ventilschaltung wenigstens zwei Ventilstellungen umfasst, wonach der Durchfluss von Flüssigkeit, insbesondere Wasser, durch die Fluidleitung zur Ableitung von Flüssigkeit, insbesondere Wasser aus dem Filtratraum blockiert werden kann oder durchgeleitet werden kann.

Gemäß dieser Ausführung ist es möglich, die im Retentatraum angereicherten Kontaminanten des wenigstens ersten Hohlfasermembranfilters in einem gleichzeitigem Vor- und Rückspülverfahren und Probenentnahme zugänglich zu machen. Unter einem *"Vorspülverfahren"* ist im Sinne der vorliegenden Ausführungsform zu verstehen, dass der Retentatraum eines Hohlfasermembranfilters mit Flüssigkeit, insbesondere Wasser durch die Fluidanschlüsse zur Zuführung und Ableitung von Flüssigkeit, insbesondere Wasser, in und aus dem Retentatraum, gespült wird. Die Kontaminanten können so aus dem Retentatraum abgeleitet werden und über Probenentnahme an der ersten Probenentnahmestelle entnommen werden und der Analyse zugeführt werden.

Unter einem *"Rückspülverfahren"* ist im Sinne der vorliegenden Ausführungsform zu verstehen, dass Flüssigkeit, insbesondere Wasser, in den Filtratraum eines Hohlfasermembranfilter geleitet wird und von dort über die Membranwandungen der Hohlfasermembranen in den Retentatraum überführt wird. Von dort kann die Flüssigkeit, insbesondere Wasser, über den Fluidanschluss und die Fluidleitung zur Ableitung von Flüssigkeit aus dem Retentatraum abgeleitet werden und zu der vorabbeschriebenen ersten oder zweiten Probenentnahmestelle geleitet werden, wo Proben entnommen werden können und auf Kontamination analysiert werden können.

Vorteilhaft können über das Rückspülverfahren auch Kontaminanten im Retentatraum des ersten Hohlfasermembranfilters freigespült werden, die in der Membranwandung der Hohlfasermembranen anhaften und über das alleinige Vorspülverfahren nur schwer freigespült werden können. Mit dem gleichzeitigem Vor- und Rückspülverfahren können damit mit einer höheren Genauigkeit die Kontaminanten im Retentatraum des ersten Hohlfasermembranfilters durch Probenentnahme und Analyse erfasst werden.

In einer weiteren Ausführungsform gemäß dem ersten Aspekt der Erfindung ist die erfindungsgemäße Vorrichtung bzw. Dialysemaschine dadurch gekennzeichnet, dass in der Vorrichtung zur Aufbereitung von Flüssigkeiten, insbesondere Wasser für die Dialyse, stromaufwärts des ersten Hohlfasermembranfilters zwischen der zweiten Fluidabzweigung an der Fluidleitung zur Zuführung von Flüssigkeit, insbesondere Wasser in den Retentatraum des ersten Hohlfasermembranfilters und dem ersten Hohlfasermembranfilter selbst im Eingriff mit der Fluidleitung zur Zuführung von Flüssigkeit, insbesondere Wasser in den Retentatraum des ersten Hohlfasermembranfilters, eine fünfte Ventilschaltung angeordnet ist, wobei die fünfte Ventilschaltung wenigstens zwei Ventilstellungen umfasst, wonach der Durchfluss von Flüssigkeit, insbesondere Wasser, durch die Fluidleitung zum Zuführen von Flüssigkeit, insbesondere Wasser in den Retentatraum des ersten Hohlfasermembranfilters, blockiert werden kann oder durchgeleitet werden kann.

Gemäß dieser Ausführungsform können Vor- und Rückspülverfahren des ersten Hohlfasermembranfilters abwechselnd durchgeführt werden, zusätzlich kann das Vor- und Rückspülverfahren auch gleichzeitig durchgeführt werden. Zur Durchführung des Vorspülverfahrens werden die Ventilstellungen der ersten und fünften Ventilschaltung in eine durchleitende Stellung gebracht und die Ventilstellungen der dritten und vierten Ventilschaltung in eine blockierende Stellung gebracht. Flüssigkeit, insbesondere Wasser, wird durch den Retentatraum geleitet, und Kontaminanten können entsprechend über Probenentnahme an der ersten Probenentnahmestelle oder der zweiten Probenentnahmestelle auf Kontamination analysiert werden.

Zur Durchführung des Rückspülverfahrens werden die dritte und fünfte Ventilschaltung in eine durchleitende Ventilstellung gebracht und Flüssigkeit, insbesondere Wasser in den Filtratraum des Hohlfasermembranfilters geleitet, über die Membranwandung in den Retentatraum überführt. Die Proben können an der ersten Probenentnahmestelle oder der dritten Probenentnahmestelle entnommen werden. Zur verbesserten Freispülung der Kontaminanten aus dem Retentatraum des ersten Hohlfasermembranfilters kann das Vor- und Rückspülverfahren abwechselnd und wiederkehrend durchgeführt werden.

Zur Durchführung des gleichzeitigen Vor- und Rückspülverfahrens werden die fünfte, dritte, vierte, und erste Ventilschaltung in eine durchleitende Ventilstellung gebracht.

In einer weiteren Ausführungsform gemäß dem ersten Aspekt der Erfindung ist die erfindungsgemäße Vorrichtung bzw. Dialysemaschine, enthaltend die Vorrichtung zur Aufbereitung von Flüssigkeiten, insbesondere Wasser für die Dialyse, dadurch gekennzeichnet, dass die Vorrichtung weiter aufweist,
wenigstens einen zweiten Hohlfasermembranfilter,
aufweisend eine Vielzahl von Hohlfasermembranen, mit einem Retentatraum und einem Filtratraum, die über semipermeable Membranwandungen der Hohlfasermembranen voneinander getrennt sind,
wobei der zweite Hohlfasermembranfilter aufweist,
einen Fluidanschluss zur Zuführung von Flüssigkeit, insbesondere Wasser in den Retentatraum des zweiten Hohlfasermembranfilters,
einen Fluidanschluss zur Ableitung von Flüssigkeit, insbesondere Wasser, aus dem Retentatraum des zweiten Hohlfasermembranfilters,
wenigstens einen Fluidanschluss zum Ableiten von Flüssigkeit, insbesondere Wasser, aus dem Filtratraum des zweiten Hohlfasermembranfilters,
und wobei die Vorrichtung weitere aufweist,
eine Fluidleitung in Flüssigkeitsverbindung mit dem Fluidanschluss zur Zuführung von Flüssigkeit, insbesondere Wasser in den Retentatraum des zweiten Hohlfasermembranfilters,
eine Fluidleitung in Flüssigkeitsverbindung mit dem Fluidanschluss zum Ableiten von Flüssigkeit, insbesondere Wasser aus dem Retentatraum des zweiten Hohlfasermembranfilters,
eine Fluidleitung in Flüssigkeitsverbindung mit dem wenigstens einen Fluidanschluss zum Ableiten von Flüssigkeit, insbesondere Wasser aus dem Filtratraum des zweiten Hohlfasermembranfilters, wobei die Vorrichtung weiter durch dadurch gekennzeichnet ist, dass die Fluidleitung in Flüssigkeitsverbindung mit dem Fluidanschluss zur Zuführung von Flüssigkeit, insbesondere Wasser in den Retentatraum des zweiten Hohlfasermembranfilters mit der Fluidleitung zur Ableitung von Flüssigkeit, insbesondere Wasser, aus dem Filtratraum des ersten Hohlfasermembranfilters flüssigkeitsverbindend verbunden ist.

Durch Einstellen der ersten Ventilschaltung und der dritten Ventilschaltung in eine blockierende Ventilstellung und Einstellen der fünften Ventilschaltung und vierten Ventilschaltung in eine durchleitende Ventilstellung, wird Flüssigkeit, insbesondere Wasser, über den Retentatraum und den Filtratraum des ersten Hohlfasermembranfilters in den Retentatraum des zweiten Hohlfasermembranfilters eingeleitet. Durch Übertritt von Flüssigkeit, insbesondere Wasser, über die Membranwandungen der Hohlfasermembranen des zweiten Hohlfasermembranfilters, vom Retentatraum in den Filtratraum des zweiten Hohlfasermembranfilters, wird die Flüssigkeit, insbesondere Wasser einer zweiten Filtration unterzogen. Die zweite Filtration erfolgt dabei vorzugsweise im "Tangential Flow" Verfahren. Dem zweiten Filter kann zusätzlich gereinigtes Wasser entnommen werden, um es in einem Dialysemodus, der als Hämodiafiltration bekannt ist, als Substituatflüssigkeit zu verwenden. Das bedeutet, dass die Substituatflüssigkeit in den Patientenkörper infundiert wird. Somit bestehen besonders hohe Anforderungen an die Reinheit und Keimfreiheit der Substituatflüssigkeit, die eine besonders empfindliche Messmethode für die Bestimmung der Kontaminanten erfordern.

Gemäß der vorliegenden Anmeldung bedeutet *"Tangential-Flow"* Filtration oder Verfahren, dass die zu filtrierende Flüssigkeit innerhalb des Hohlfasermembranfilters entlang der Membranoberfläche der Hohlfasermembranen geleitet wird. Ein Teil der Flüssigkeit tritt dabei als Filtrat durch die Membranwand auf Filtratseite über. Ein weiterer Teil der Flüssigkeit verbleibt im Retentatraum diesseits der Membranwand und wird als sogenanntes Retentat aus dem Filter abgeleitet. In dieser Anordnung wird demnach Flüssigkeit in den Retentatraum des zweiten Hohlfasermembranfilters eingeleitet und ein Teil der Flüssigkeit über den Fluidanschluss zur Ableitung von Flüssigkeit aus dem Retentatraum abgeleitet. Ein weiterer Teil tritt über die Membranwandung der Hohlfasermembranen in den Filtratraum des zweiten Hohlfasermembranfilters über und wir über den Fluidanschluss und Fluidleitung zur Ableitung von Flüssigkeit aus dem Filtratraum des zweiten Hohlfasermembranfilters abgeleitet.

Vorteilhafterweise kann an einer Entnahmestelle, die in Flüssigkeitsverbindung mit der Fluidleitung zur Ableitung von Flüssigkeit, insbesondere Wasser, aus dem Filtratraum des zweiten Hohlfasermembranfilters steht aufbereitete Flüssigkeit, die über zwei Filtrationsstufen aufbereitete wurde, entnommen werden und für die Herstellung von Substitutionsflüssigkeit bereitgestellt werden.

In einer weiteren Ausführungsform gemäß dem ersten Aspekt der Erfindung ist die erfindungsgemäße Vorrichtung bzw. Dialysemaschine, enthaltend die Vorrichtung zur Aufbereitung einer Flüssigkeiten, insbesondere Wasser, dadurch gekennzeichnet, dass in der Fluidleitung zwischen Fluidanschluss zur Ableitung von Flüssigkeit, insbesondere Wasser aus dem Retentatraum des zweiten Hohlfasermembranfilters eine vierte Fluidabzweigung angeordnet ist, von der eine zweite abzweigende Fluidleitung abzweigt, weiterhin dadurch gekennzeichnet, dass stromabwärts der vierten Fluidabzweigung eine sechste Ventilschaltung im Eingriff mit der der Fluidleitung zum Ableiten von Flüssigkeit, insbesondere Wasser, aus dem Retentatraum des zweiten Hohlfasermembranfilters angeordnet ist,
wobei die sechste Ventilschaltung wenigstens zwei Ventilstellungen umfasst, wonach der Durchfluss von Flüssigkeit, insbesondere Wasser, durch die Fluidleitung zum Ableiten von Flüssigkeit, insbesondere Wasser, aus dem Retentatraum des zweiten Hohlfasermembranfilters, blockiert werden kann oder durchgeleitet werden kann.
weiterhin dadurch gekennzeichnet, dass die zweite abzweigende Fluidleitung mit einer vierten Probeentnahmestelle in Flüssigkeitsverbindung steht.

Durch Einstellen der sechsten Ventilschaltung in eine blockierende Stellung, wird die aus dem Retentatraum des zweiten Hohlfasermembranfilters abgeleitete Flüssigkeit, insbesondere Wasser, über die zweite abzweigende Leitung zur vierten Probeentnahmestelle geleitet. An der vierten Probenentnahmestelle können Proben entnommen werden und der Analyse zugeführt werden. Gemäß der Vorrichtung dieser Ausführungsform kann der Retentatraum des zweiten Hohlfasermembranfilters auf Kontamination untersucht werden. Insbesondere kann dadurch auch untersucht werden, ob die Flüssigkeit aus der ersten Filtrationsstufe, die durch "dead-end" Filtration über Retentatraum und Filtratraum des ersten Hohlfasermembranfilters in den Retentatraum des zweiten Hohlfasermembranfilters übergeleitet wird mit Kontaminanten belastet ist. Die Feststellung von Kontaminanten in Proben, die an der Probenentnahmestelle entnommen wurden lässt somit Rückschlüsse auf die Qualität der Filtration am ersten Hohlfasermembranfilter zu.

In einer weiteren Ausführungsform gemäß der vorhergehenden Ausführung gemäß dem ersten Aspekt der Erfindung, ist die erfindungsgemäße Vorrichtung bzw. Dialysemaschine, enthaltend die Vorrichtung zur Aufbereitung einer Flüssigkeit, insbesondere Wasser für die Dialyse, dadurch gekennzeichnet, dass
von der Fluidleitung zur Zuführung von Flüssigkeit, insbesondere Wasser, in den Retentatraum des ersten Hohlfasermembranfilters stromaufwärts des ersten Hohlfasermembranfilters (F1) eine zweite Fluidverzweigung angeordnet ist,
weiter dadurch gekennzeichnet, dass die Vorrichtung an der Fluidleitung zur Ableitung von Flüssigkeit, insbesondere Wasser, aus dem Filtratraum des zweiten Hohlfasermembranfilters eine fünfte Fluidverzweigung aufweist,
weiter dadurch gekennzeichnet, dass eine Fluidleitung in Flüssigkeitsverbindung mit der zweiten Fluidverzweigung an der Fluidleitung zur Zuführung von Flüssigkeit, insbesondere Wasser, in den Retentatraum des ersten Hohlfasermembranfilters und der fünften Fluidverzweigung an der Fluidleitung zur Ableitung von Flüssigkeit, insbesondere Wasser, aus dem Filtratraum des zweiten Hohlfasermembranfilters angeordnet ist,
weiter dadurch gekennzeichnet, dass im Eingriff mit der Fluidleitung zwischen zweiten Fluidverzweigung an der Fluidleitung zur Zuführung von Flüssigkeiten, insbesondere Wasser in den Retentatraum des ersten Hohlfasermembranfilters und der fünften Fluidverzweigung an der Fluidleitung zur Ableitung von Flüssigkeit, insbesondere Wasser aus dem Filtratraum des zweiten Hohlfasermembranfilters eine dritte Ventilschaltung angeordnet ist,
wobei die dritte Ventilschaltung wenigstens zwei Ventilstellungen umfasst, wonach der der Durchfluss von Flüssigkeit, insbesondere Wasser, durch die Fluidleitung zwischen der zweiten Fluidabzweigung und der fünften Fluidabzweigung blockiert werden kann oder durchgeleitet werden kann,
weiter dadurch gekennzeichnet, dass der Fluidanschluss zum Zuführen von Flüssigkeit, insbesondere Wasser, in den Retentatraum des zweiten Hohlfasermembranfilters über eine Fluidleitung mit dem Fluidanschluss zum Ableiten von Flüssigkeit, insbesondere Wasser, aus dem Filtratraum des ersten Hohlfasermembranfilters in Flüssigkeitsverbindung besteht.
weiter dadurch gekennzeichnet, dass stromaufwärts des Filters zwischen der zweiten Fluidverzweigung im Eingriff mit der Fluidleitung zur Zuführung von Flüssigkeit, insbesondere Wasser, in den Retentatraum und dem ersten Hohlfasermembranfilter Filter eine fünfte Ventilschaltung angeordnet ist,
wobei die fünfte Ventilschaltung wenigstens zwei Ventilstellungen umfasst, wonach der der Durchfluss von Flüssigkeit, insbesondere Wasser, Fluidleitung zur Zuführung von Flüssigkeit, insbesondere Wasser, in den Retentatraum blockiert werden kann oder durchgeleitet werden kann.

Ein Vorteil dieser Ausführungsform besteht darin, dass der Retentatraum des zweiten Hohlfasermembranfilters nach einem Vorspülverfahren durch Probenentnahme und Analyse auf Kontamination überprüft werden kann. Dabei sind die dritte Ventilschaltung und die erste Ventilschaltung in eine blockierende Ventilstellung gebracht und die fünfte Ventilschaltung ist in eine durchleitende Ventilstellung gebracht. Zu Überprüfung auf Kontamination wird Flüssigkeit, insbesondere Wasser in den Retentatraum des ersten Hohlfasermembranfilters eingeleitet, im "dead-end" Verfahren über die Membranwandung der Hohlfasermembranen des ersten Hohlfasermembranfilters geführt, und in den Retentatraum des zweiten Hohlfasermembranfilters eingeleitet. Von der Flüssigkeit, die anschließend aus dem Retentatraum des zweiten Hohlfasermembranfilters abgeleitet wird können Proben entnommen werden und auf Kontamination untersucht werden.

In einer weiteren Alternative kann die fünfte Ventilschaltung in eine blockierende Ventilstellung gebracht werden und die dritte Ventilschaltung kann in eine durchleitende Ventilstellung gebracht werden. Gemäß dieser Anordnung der Ventilschaltungen wird Flüssigkeit, insbesondere Wasser, in den Filtratraum des zweiten Hohlfasermembranfilters eingeleitet und über die Membranwandung in den Retentatraum des zweiten Hohlfasermembranfilters gemäß eines Rückspülverfahrens geführt. Dadurch können an der Membranwandung der Hohlfasermembranen des zweiten Hohlfasermembranfilters anhaftende Kontaminanten freigespült werden und über Probenentnahme z.B. an der vierten Probenentnahmestelle auf Kontamination analysiert werden.

In einer der vorliegend beschriebenen Ausführungsformen weist die Vorrichtung bzw. Dialysemaschine eine Analysevorrichtungen auf, mit der an den Probenentnahmestellen an einer gewonnenen Probe ein Analysewert bezüglich der Konzentration von Kontaminanten in einer jeweiligen Probe gewonnen werden kann. Insbesondere ist eine solche Analysevorrichtung an der Probenentnahmestelle stromabwärts der ersten Ventilschaltung in Flüssigkeitsverbindung mit der Fluidleitung zum Ableiten von Flüssigkeit, insbesondere Wasser, aus dem Retentatraum des ersten Hohlfasermembranfilters angeordnet. Zusätzlich kann die Vorrichtung bzw. Dialysemaschine, enthaltend die in einer Ausführungsform beschriebene Vorrichtung, so konfiguriert sein, dass die Mittel zur Bestimmung des Volumens der vom Retentatraum in den Filtratraum des ersten Hohlfasermembranfilters überführten Flüssigkeit, insbesondere Wasser, aufweist. Zusätzlich kann die Vorrichtung bzw. Dialysemaschine, die in einer Ausführungsform beschriebene Vorrichtung, so konfiguriert sein, dass sie Mittel zur Bestimmung des Volumens an Flüssigkeit, insbesondere Wasser, mit der die Kontaminanten zu der Probenentnahmestelle gespült werden, aufweist. Zusätzlich kann die Vorrichtung bzw. Dialysemaschine eine elektronische Auswerteeinheit aufweisen.

Die Auswerteeinheit ist so konfiguriert, dass sie aus dem Analysewert bezüglich der Konzentration von Kontaminanten in einer jeweiligen Probe, dem Volumen der vom Retentatraum in den Filtratraum des ersten Hohlfasermembranfilters überführten Flüssigkeit, insbesondere Wasser und dem Volumen an Flüssigkeit, insbesondere Wasser, mit der die Kontaminanten zu der Probenentnahmestelle gespült werden, einen Umrechnungsfaktor ermittelt. Die Auswerteeinheit ist somit geeignet, die Menge der Flüssigkeit, insbesondere Wasser für die Dialyse zu bestimmen, die vom Retentatraum in den Filtratraum des ersten Hohlfasermembranfilters überführt wird bevor ein Spülvorgang für die Analyse einsetzt. Der Umrechnungsfaktor stellt somit einen Wert für die Aufkonzentrierung der Kontaminanten bereit. Wird Analysewert bezüglich der Konzentration von Kontaminanten in einer jeweiligen Probe beispielsweise in KBE/ml (keimbildende Einheiten pro Milliliter) angegeben, so kann über den Umrechnungsfaktor ein Wert für eine Kontamination für die für die Vorrichtung bzw. Dialysemaschine bereitgestellte Flüssigkeit, insbesondere Wasser, ermittelt werden. Es können auch nichtganzzahlige Werte ermittelt werden. Insbesondere können auch besonders kleine Werte sicher ermittelt werden, die durch direkte Analyse der bereitgestellten Flüssigkeit, insbesondere Wasser nicht zu ermitteln sind.

In einem zweiten Aspekt betrifft die Erfindung ein Verfahren zur Überwachung der Aufbereitung von Flüssigkeiten, insbesondere zur Aufbereitung von Wasser für die Dialyse, in einer Vorrichtung bzw. Dialysemaschine aufweisend die Schritte
Bereitstellen einer Vorrichtung nach dem ersten Aspekt der Erfindung,
Einstellen der ersten Ventilschaltung und gegebenenfalls der zweiten Ventilschaltung in eine blockierende Stellung,
Einleiten von Flüssigkeit, insbesondere Wasser, in den Retentatraum des Hohlfasermembranfilters,
Filtrieren der Flüssigkeit, insbesondere Wasser über die Membranwandung der Hohlfasermembranen von dem Retentatraum in den Filtratraum des ersten Hohlfasermembranfilters und auffangen von der Flüssigkeit, insbesondere Wasser im Filtratraum, wobei bei der Filtration Kontaminanten im Rohwasser von der Hohlfasermembran zurückgehalten werden,
Ableiten der Flüssigkeit, insbesondere des Wassers, aus dem Filtratraum,
Einstellen der ersten Ventilschaltung in eine durchleitende Stellung, nachdem eine vorherbestimmte Menge an Flüssigkeit, insbesondere Wasser durch Filtration in den Filtratraum überführt wurde,
wobei die gegebenenfalls zweite Ventilschaltung in einer blockierenden Ventilstellung verbleibt,
weiteres Einleiten von Flüssigkeit, insbesondere Wasser in den Retentatraum des ersten Hohlfasermembranfilters,
dadurch gekennzeichnet, dass an der ersten Probenentnahmestelle stromabwärts der ersten Ventilschaltung Proben entnommen werden und die Proben auf Kontamination analysiert werden
oder gegebenenfalls
Einstellen der ersten Ventilschaltung in eine blockierende Ventilstellung, und Einstellen der zweiten Ventilschaltung in eine durchleitende Ventilstellung nachdem eine vorherbestimmte Menge an Flüssigkeit, insbesondere Wasser, durch Filtration in den Filtratraum des ersten Hohlfasermembranfilters überführt wurde,
weiteres Einleiten von Flüssigkeit, insbesondere Wasser, in den Retentatraum des ersten Hohlfasermembranfilters,
dadurch gekennzeichnet, dass an der zweiten Probenentnahmestelle stromabwärts der ersten Fluidverzweigung Proben entnommen werden und die Proben auf Kontamination analysiert werden.

Gemäß des erfindungsgemäßen Verfahrens kann eine große Menge an Flüssigkeit, insbesondere Wasser, filtriert werden und damit eine Anreicherung von Kontaminanten korrespondierend zur Menge des filtrierten Flüssigkeit, insbesondere Wasser, erzielt werden. Infolge dessen kann nach dem beschriebenen Verfahren auf die Ausgangskontamination der Flüssigkeit, insbesondere Wasser geschlossen werden.

Unter den Begriffen "Filtration", oder "filtrieren" wird im Sinne der vorliegenden Anmeldung das Überleiten von Flüssigkeit über die Membranwandung der Hohlfasermembranen des Hohlfasermembranfilters vom Retentatraum in den Filtratraum verstanden, wobei Bestandteile, insbesondere Kontaminanten, die in der Flüssigkeit, insbesondere Wasser, vorliegen, von der Membranwand zurückgehalten werden.

In einer weitere Ausführungsform des zweiten Aspekts betriff die Erfindung daher ein Verfahren zur Überwachung der Aufbereitung von Flüssigkeit, insbesondere Wasser für Dialyse, wobei die vorherbestimmte Menge an Flüssigkeit, insbesondere Wasser, die durch Filtration in den Filtratraum des ersten Hohlfasermembranfilters überführt wurde mindestens 20 l, oder mehr, bevorzugt mindestens 100 I oder mehr, weiter bevorzugt mindestens 200 I oder mehr beträgt.

In einer weiteren Ausführungsform gemäß dem zweiten Aspekt betrifft die Erfindung ein Verfahren zur Überwachung der Aufbereitung von Flüssigkeit, insbesondere Wasser für die Dialyse, aufweisend die Schritte,

Bereitstellen einer Vorrichtung nach einer Ausführungsform des ersten Aspekts der Erfindung
Einstellen der ersten Ventilschaltung in eine blockierende Ventilstellung,
Einstellen der dritten Ventilschaltung in eine blockierende Ventilstellung,
Einstellen der vierten Ventilschaltung in eine durchleitende Ventilstellung,
Einleiten von Flüssigkeit, insbesondere Wasser, in den Retentatraum des ersten Hohlfasermembranfilters,
Filtrieren der Flüssigkeit, insbesondere Wasser, über die Membranwandung der Hohlfasermembranen des ersten Hohlfasermembranfilters, wobei bei der Filtration Kontaminanten der Flüssigkeit, insbesondere Wasser, von der Membranwandung der Hohlfasermembranen zurückgehalten werden,
Überleiten von Flüssigkeit, insbesondere Wasser, aus dem Filtratraum des ersten Hohlfasermembranfilters in den Retentatraum des zweiten Hohlfasermembranfilters,
Filtrieren der Flüssigkeit, insbesondere Wassers, im Retentatraum des zweiten Hohlfasermembranfilters über die Membranwandung der Hohlfasermembranen des zweiten Hohlfasermembranfilters, wobei bei der Filtration Kontaminanten der Flüssigkeit von der Membranwandung der Hohlfasermembranen des zweiten Hohlfasermembranfilters zurückgehalten werden,
Ableiten von Flüssigkeit, insbesondere Wasser, aus dem Filtratraum des zweiten Hohlfasermembranfilters,
gegebenenfalls Entnahme von Flüssigkeit, insbesondere Wasser, oder Proben an einer Entnahmestelle, die mit der Fluidleitung zur Ableitung von Flüssigkeit, insbesondere Wasser aus dem Filtratraum des zweiten Hohlfasermembranfilters verbunden ist,
anschließendes Einstellen der ersten Ventilschaltung in eine durchleitende Stellung,
Einstellen der dritten Ventilschaltung in eine durchleitende Ventilstellung,
Einstellen der vierten Ventilschaltung in eine blockierende Ventilstellung,
Einstellen der ersten Ventilstellung in eine durchleitende Stellung,
nachdem eine vorherbestimmte Menge an Flüssigkeit, insbesondere Wasser, durch Filtration in den Filtratraum überführt wurde
Filtrieren der Flüssigkeit, insbesondere Wasser im Filtratraum des ersten Hohlfasermembranfilters über die Membranwandung der Hohlfasermembranen des ersten Hohlfasermembranfilters in den Retentatraum des ersten Hohlfasermembranfilters, wobei bei der Filtration Kontaminanten aus dem Retentatraum des ersten Hohlfasermembranfilters freigespült werden,
Ableiten von Flüssigkeit aus dem Retentatraum des ersten Hohlfasermembranfilters,
dadurch gekennzeichnet, dass an der ersten Probenentnahmestelle stromabwärts der ersten Ventilschaltung Proben entnommen werden und die Proben auf Kontamination analysiert werden.

Das Verfahren weist den Vorteil des gleichzeitigen Spülens von Retentatraum und Filtratraum des ersten Hohlfasermembranfilters im gleichzeitigen Vor- und Rückspülverfahren nach Anreicherung von Kontaminanten im ersten Hohlfasermembranfilter, wie vorab beschrieben wurde auf.

In einer weitere Ausführungsform gemäß dem zweiten Aspekt betrifft die Erfindung ein Verfahren zur Überwachung der Aufbereitung von Flüssigkeit, insbesondere Wasser für die Dialyse, aufweisend die Schritte,
Bereitstellen einer Vorrichtung nach einer Ausführungsform des ersten Aspekts der Erfindung
Einstellen der ersten Ventilschaltung in eine blockierende Ventilstellung,
Einstellen der fünften Ventilschaltung in eine durchleitende Ventilstellung
Einstellen der dritten Ventilschaltung in eine blockierende Ventilstellung,
Einstellen der vierten Ventilschaltung in eine durchleitende Ventilstellung,
Einleiten von Flüssigkeit, insbesondere Wasser, in den Retentatraum des ersten Hohlfasermembranfilters,
Filtrieren der Flüssigkeit, insbesondere Wasser, über die Membranwandung der Hohlfasermembranen des ersten Hohlfasermembranfilters, wobei bei der Filtration Kontaminanten der Flüssigkeit, insbesondere Wasser, von der Membranwandung der Hohlfasermembranen zurückgehalten werden,
Überleiten von Flüssigkeit, insbesondere Wasser, aus dem Filtratraum des ersten Hohlfasermembranfilters in den Retentatraum des zweiten Hohlfasermembranfilters,
Filtrieren der Flüssigkeit, insbesondere Wassers, im Retentatraum des zweiten Hohlfasermembranfilters über die Membranwandung der Hohlfasermembranen des zweiten Hohlfasermembranfilters, wobei bei der Filtration Kontaminanten der Flüssigkeit von der Membranwandung der Hohlfasermembranen des zweiten Hohlfasermembranfilters zurückgehalten werden,
Ableiten von Flüssigkeit, insbesondere Wasser, aus dem Filtratraum des zweiten Hohlfasermembranfilters,
gegebenenfalls Entnahme von Flüssigkeit, insbesondere Wasser oder Proben an der Entnahmestelle, die mit der Fluidleitung zur Ableitung von Flüssigkeit, insbesondere Wasser aus dem Filtratraum des zweiten Hohlfasermembranfilters verbunden ist und gegebenenfalls Analysieren der Proben auf Kontamination,
anschließendes Einstellen der ersten Ventilschaltung in eine durchleitende Stellung,
Einstellen der fünften Ventilschaltung in eine blockierende Ventilstellung
Einstellen der dritten Ventilschaltung in eine durchleitende Ventilstellung,
Einstellen der vierten Ventilschaltung in eine blockierende Ventilstellung,
nachdem eine vorherbestimmte Menge an Flüssigkeit, insbesondere Wasser, durch Filtration in den Filtratraum überführt wurde
Filtrieren der Flüssigkeit, insbesondere Wasser im Filtratraum des ersten Hohlfasermembranfilters über die Membranwandung der Hohlfasermembranen des ersten Hohlfasermembranfilters in den Retentatraum des ersten Hohlfasermembranfilters, wobei bei der Filtration Kontaminanten aus dem Retentatraum des ersten Hohlfasermembranfilters freigespült werden,
Ableiten von Flüssigkeit aus dem Retentatraum des ersten Hohlfasermembranfilters,
dadurch gekennzeichnet, dass an der ersten Probenentnahmestelle stromabwärts der ersten Ventilschaltung Proben entnommen werden und die Proben auf Kontamination analysiert werden.

Vor- und Rückspülung des ersten Hohlfasermembranfilters gemäß dem beschriebenen Verfahren dieser Ausführungsform kann nach Anreicherung von Kontaminanten abwechselnd oder gleichzeitig und wiederholend durchgeführt werden. Das Verfahren weist den Vorteil eines verbesserten Freispülens des Hohlfasermembranfilters von Kontamination auf, was insbesondere bei geringen Kontaminationsgrad vorteilhaft ist.

In einer weiteren Ausführungsform gemäß dem zweiten Aspekt betrifft die Erfindung ein Verfahren zur Überwachung der Aufbereitung von Flüssigkeit, insbesondere Wasser für die Dialyse, aufweisend die Schritte,
Bereitstellen einer Vorrichtung nach einer Ausführungsform des ersten Aspekts der Erfindung
Einstellen der ersten Ventilschaltung in eine blockierende Stellung,
Einstellen der sechsten Ventilschaltung in eine durchleitende Stellung
Einleiten von Flüssigkeit, insbesondere Wasser, in den Retentatraum des ersten Hohlfasermembranfilters,
Filtrieren von Flüssigkeit, insbesondere Wasser, über die Membranwandung der Hohlfasermembranen vom Retentatraum in den Filtratraum des ersten Hohlfasermembranfilters und Auffangen von Flüssigkeit im Filtratraum, wobei bei der Filtration Kontaminanten der Flüssigkeit, insbesondere Wasser, von der Hohlfasermembran zurückgehalten werden,
Überleiten von Flüssigkeit, insbesondere Wasser, vom Filtratraum des ersten Hohlfasermembranfilters in den Retentatraum des zweiten Hohlfasermembranfilters,
Einstellen der sechsten Ventilschaltung in eine blockierende Stellung, nachdem eine vorherbestimmte Menge an Flüssigkeit, insbesondere Wasser, durch Filtration in den Filtratraum des ersten Hohlfasermembranfilters überführt wurde,
weiteres Einleiten von Flüssigkeit, insbesondere Wasser, in den Retentatraum des ersten Hohlfasermembranfilters, Filtrieren von Flüssigkeit, insbesondere Wasser, in den Filtratraum des ersten Hohlfasermembranfilters, Überleiten von Flüssigkeit, insbesondere Wasser, vom Filtratraum des ersten Hohlfasermembranfilters in den Retentatraum des zweiten Hohlfasermembranfilters,
dadurch gekennzeichnet, dass an der vierten Probenentnahmestelle (P4) Proben entnommen werden und die Proben auf Kontamination analysiert werden.

Das Verfahren weist den Vorteil auf, dass der Retentatraum des zweiten Hohlfasermembranfilters nach Anreicherung von Kontaminanten im ersten Hohlfasermembranfilter und oder/ zweiten Hohlfasermembranfilter, wie vorab beschrieben wurde, auf Kontamination untersucht werden kann.

In einer weiteren Ausführungsform gemäß dem zweiten Aspekt betrifft die Erfindung ein Verfahren zur Überwachung der Aufbereitung von Flüssigkeit, insbesondere Wasser für die Dialyse, aufweisend die Schritte,
Bereitstellen einer Vorrichtung einer Ausführungsform gemäß dem ersten Aspekt der Erfindung,
Einstellen der ersten Ventilschaltung in eine blockierende Stellung,
Einstellen der fünften Ventilschaltung, in eine durchleitende Stellung,
Einstellen der dritten Ventilschaltung in eine blockierende Stellung,
Einstellen der sechsten Ventilschaltung in eine durchleitende Stellung,
Einleiten von Flüssigkeit, insbesondere Wasser, in den Retentatraum des Hohlfasermembranfilters,
Filtrieren der Flüssigkeit, insbesondere Wasser über die Membranwandung der Hohlfasermembranen vom Retentatraum in den Filtratraum des ersten Hohlfasermembranfilters und auffangen von Flüssigkeit, insbesondere Wasser im Filtratraum, wobei bei der Filtration Kontaminanten der Flüssigkeit, insbesondere Wasser, von der Hohlfasermembran zurückgehalten werden,
Überleiten von Flüssigkeit, insbesondere Wasser aus dem Filtratraum des ersten Hohlfasermembranfilters in den Retentatraum des zweiten Hohlfasermembranfilters,
Filtrieren von Flüssigkeit, insbesondere Wasser, über die Membranwandung der Hohlfasermembranen vom Retentatraum in den Filtratraum des zweiten Hohlfasermembranfilters und auffangen von Flüssigkeit, insbesondere Wasser, im Filtratraum des zweiten Hohlfasermembranfilters, wobei bei der Filtration Kontaminanten im Rohwasser von der Hohlfasermembran zurückgehalten werden,
Ableiten von Wasser aus dem Filtratraum des zweiten Hohlfasermembranfilters und gegebenenfalls Entnahme von Flüssigkeit, insbesondere Wasser, oder Proben an der Entnahmestelle, die mit der Fluidleitung zur Ableitung von Flüssigkeit, insbesondere Wasser aus dem Filtratraum des zweiten Hohlfasermembranfilters verbunden ist und gegebenenfalls Analysieren der Proben auf Kontamination,
anschließendes Einstellen der fünften Ventilschaltung, in eine blockierende Stellung,
Einstellen der dritten Ventilschaltung in eine durchleitende Stellung,
Einstellen der sechsten Ventilschaltung in eine blockierende Stellung, nachdem eine vorherbestimmte Menge an Flüssigkeit, insbesondere Wasser, durch Filtration in den Filtratraum überführt wurde,
Einleiten von Flüssigkeit, insbesondere Wasser in den Filtratraum des zweiten Hohlfasermembranfilters, filtrieren von Flüssigkeit, insbesondere Wassers über die Membranwandung der Hohlfasermembranen vom Filtratraum in den Retentatraum des zweiten Hohlfasermembranfilters und auffangen von Flüssigkeit, insbesondere Wasser im Retentatraum des zweiten Hohlfasermembranfilters, wobei bei der Filtration Kontaminanten aus dem Retentatraum des zweiten Hohlfasermembranfilters, die von den Hohlfasermembran zurückgehalten wurden freigespült werden,
dadurch gekennzeichnet, dass an der vierten Probenentnahmestelle Proben entnommen werden und die Proben auf Kontamination analysiert werden.

Das Verfahren weist den Vorteil auf, dass der Retentatraum des zweiten Hohlfasermembranfilters nach Anreicherung von Kontaminanten im ersten Hohlfasermembranfilter und oder / zweiten Hohlfasermembranfilter, wie vorab beschrieben wurde, auf Kontamination untersucht, indem der zweite Hohlfasermembranfilter in einem Rückspülverfahren gespült wird. Über das Rückspülverfahren kann mit größerer Genauigkeit Kontaminanten nach Anreicherung freigespült werden und analysiert werden, was bei geringeren Kontaminationen vorteilhaft ist.

In einer weiteren Ausführungsform gemäß dem zweiten Aspekt betrifft die Erfindung ein Verfahren zur Überwachung der Aufbereitung von Flüssigkeit, insbesondere Wasser für die Dialyse, aufweisend einen Schritt, bei dem ein Umrechnungsfaktor ermittelt wird, mit dem ein Analysewert der für die Vorrichtung bzw. Dialysemaschine bereitgestellten Flüssigkeit, insbesondere Wasser angegeben werden kann. Im Einzelnen ist vorgesehenen, dass an einer der Probenentnahmestellen eine Probe entnommen wird und über eine Analysevorrichtung ein Analysewert bezüglich der Konzentration von Kontaminanten ermittelt wird, wobei weiterhin das Volumen von Flüssigkeit, insbesondere Wasser, ermittelt wird, dass vom Retentatraum in den Filtratraum des ersten Hohlfasermembranfilters überführt wird, wobei weiterhin das Volumens an Flüssigkeit, insbesondere Wasser, mit der die Kontaminanten zu der Probenentnahmestelle gespült werden, bestimmt wird, dadurch gekennzeichnet, dass über eine elektronische Auswerteeinheit, die so konfiguriert, dass sie aus dem Analysewert bezüglich der Konzentration von Kontaminanten in einer jeweiligen Probe, dem Volumen der vom Retentatraum in den Filtratraum des ersten Hohlfasermembranfilters überführten Flüssigkeit, insbesondere Wasser, und dem Volumen an Flüssigkeit, insbesondere Wasser, mit der die Kontaminanten zu der Probenentnahmestelle gespült werden, ein Umrechnungsfaktor ermittelt wird, und gegebenenfalls ein Wert für eine Kontamination für die für die Vorrichtung bzw. Dialysemaschine bereitgestellte Flüssigkeit, insbesondere Wasser, ermittelt wird.

Es ist somit möglich, die Menge des Wassers für die Dialyse zu bestimmen, die den Filter passiert, bevor ein Spülvorgang für die Analyse einsetzt. Der Umrechnungsfaktor stellt somit einen Wert für das Maß der Aufkonzentrierung der Kontaminanten bereit. Wird Analysewert bezüglich der Konzentration von Kontaminanten in einer jeweiligen Probe beispielsweise in KBE/ml (keimbildende Einheiten pro Milliliter) angegeben, so kann über den Umrechnungsfaktor ein Wert für eine Kontamination für die für die Vorrichtung bzw. Dialysemaschine bereitgestellte Flüssigkeit, insbesondere Wasser, ermittelt werden. Es können auch nichtganzzahlige Werte ermittelt werden. Insbesondere können auch besonders kleine Werte sicher ermittelt werden, die durch direkte Analyse der bereitgestellten Flüssigkeit, insbesondere Wasser, nicht zu ermitteln sind.

### BESCHREIBUNG DER ERFINDUNG ANHAND DER FIGUREN

Im Folgenden werden weitere Einzelheiten und Ausführungsformen der Erfindung anhand der Figuren beschrieben. Zur Verdeutlichung der Erfindung ist die erfindungsgemäße Dialysemaschine nicht mit dargestellt. Die in den Figuren dargestellten Vorrichtungen sind typischerweise innerhalb der Dialysemaschine angeordnet.

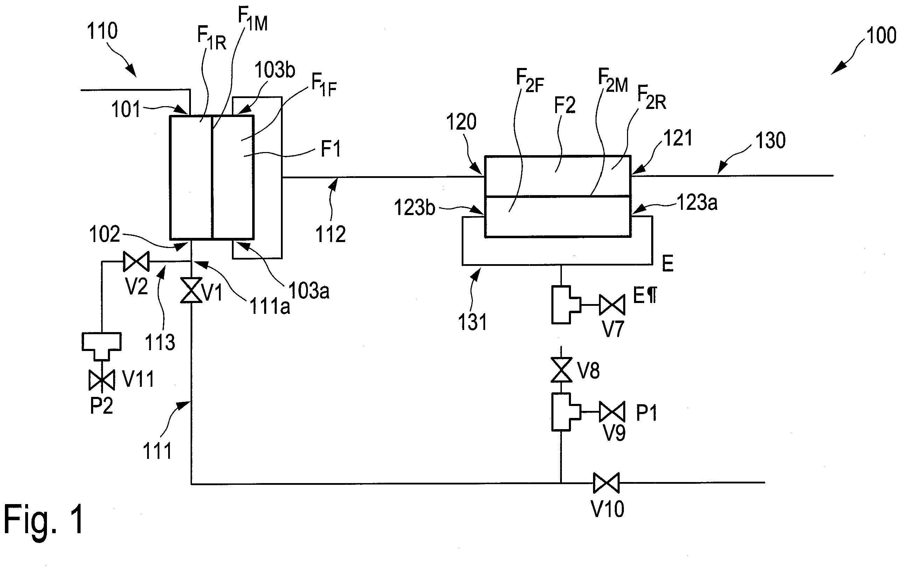

Fig. 1 zeigt eine schematische Abbildung einer erfindungsgemäßen Ausführungsform der Vorrichtung nach Anspruch 1. In der Fig. 1 bezeichnet 100 die Vorrichtung zur Aufbereitung von Flüssigkeiten, insbesondere Wasser für die Dialyse. F1 bezeichnet einen Hohlfasermembranfilter mit einer Vielzahl von Hohlfasermembranen. Schematisch ist der Retentatraum F_{1R} und der Filtratraum F_{1F} des Hohlfasermembranfilters F1, die durch die Membranwandung F_{1M} der Vielzahl von Hohlfasermembranen (nicht in der Abbildung gezeigt) gebildet werden. Weiterhin zeigt Fig. 1 den Fluidanschluss 101 und die Fluidleitung 110 zur Zuführung von Flüssigkeit, insbesondere Wasser in den Retentatraum F_{1R} des ersten Hohlfasermembranfilters F1. Weiter zeigt Fig.1 Fluidanschluss 102 und Fluidleitung 111, zur Ableitung von Flüssigkeit, insbesondere Wasser aus dem Retentatraum F_{1R} des Hohlfasermembranfilters, wenigstens einen Anschluss 103a bzw. 103b zum Ableiten von Flüssigkeit, insbesondere Wasser aus dem Filtratraum F_{1F} des ersten Hohlfasermembranfilters, eine Fluidleitung 112 zur Ableitung von Flüssigkeit, insbesondere Wasser, aus dem Filtratraum, eine erste Ventilschaltung V1 im Eingriff mit der Fluidleitung zur Ableitung von Flüssigkeit, insbesondere Wasser, aus dem Retentatraum F_{1R} des ersten Hohlfasermembranfilters F1 und stromabwärts der ersten Ventilschaltung eine Probeentnahmestelle P1.

Fig.1 zeigt weitere Einzelheiten der Vorrichtung zur Aufbereitung von Flüssigkeiten, insbesondere Wasser für die Dialyse. Fig.1 zeigt demnach einen zweiten Hohlfasermembranfilter F2 mit einem Retentatraum F_{2R}, einem Filtratraum F_{2F}, die durch die Membranwandung der Vielzahl von Hohlfasermembranen gebildet werden. Weiterhin zeigt die Fig. 1 einen Fluidanschluss 120 zur Zuführung von Flüssigkeit, insbesondere Wasser, in den Retentatraum F_{2R} des zweiten Hohlfasermembranfilters F2, den Fluidanschluss 121 und Fluidleitung 130 zur Ableitung von Flüssigkeit, insbesondere Wasser aus dem Retentatraum F_{2R} des zweiten Hohlfasermembranfilters F2, wenigstens einen Fluidanschluss 103a/ 103b und eine Fluidleitung 131 zur Ableitung von Flüssigkeit, insbesondere Wasser, aus dem Filtratraum F_{2F} des zweiten Hohlfasermembranfilters F2, eine Entnahmestelle E, sowie siebte V7, achte V8, neunte V9, zehnte V10 Ventilschaltungen zur weiteren Unterstützung der Aufbereitung von Flüssigkeiten, insbesondere Wasser in der erfindungsgemäßen Vorrichtung.

Gemäß dem erfindungsgemäßen Verfahren wird Flüssigkeit, insbesondere Wasser, aufbereitet, indem Flüssigkeit, insbesondere Wasser über die Fluidleitung 110 und den Fluidanschluss 101 in den Retentatraum F_{1R} des ersten Hohlfasermembranfilters eingeleitet wird. Die Stellung des ersten Ventils V1 ist auf eine den Durchfluss dabei auf eine blockierende Ventilstellung eingestellt. Das Einleiten von Wasser kann über Pumpenmittel erfolgen, die in der vorliegenden Fig. 1 nicht abgebildet sind. Die Flüssigkeit wird über die Membranwandung F_{1M} in den Filtratraum F_{1F} des ersten Hohlfasermembranfilters F1 überführt und über die Fluidanschlüsse 103a/b, 120 und Fluidleitung 112 in den Retentatraum F_{2R} des zweiten Hohlfasermembranfilters F2 übergeleitet, um gegebenenfalls in einer weiteren Filtration im "Tangential-Flow" Verfahren eine aufbereitete Flüssigkeit, insbesondere Wasser zur Herstellung einer Dialysierflüssigkeit, bereitzustellen.

Der Retentatraum F_{1R} des ersten Hohlfasermembranfilters wird auf Kontamination geprüft, indem die Stellung der Ventilschaltung V1 in eine durchleitende Stellung gebracht wird. Die angereicherten Kontaminanten im Retentatraum F_{1R} des ersten Hohlfasermembranfilters F1 können auf diese Weise über die Fluidleitung 111 abgeleitet werden. An der Probenentnahmestelle P1 können Proben entnommen werden und der Analyse auf Kontamination zugeführt werden, so dass eine Aussage über die Kontamination des Retentatraumes F_{1R} des ersten Hohlfasermembranfilters F1 gemacht werden kann. Es kann eine hier nicht gezeigte Auswerteeinheit in der Vorrichtung bzw. Dialysemaschine vorgesehen sein, die das filtrierte Volumen überwacht. Da auch das Retentatspülvolumen überwacht werden kann, ist es somit möglich, einen Umrechnungsfaktor auszugeben, mit dem die initiale Keimkonzentration des ungefilterten Wassers, beispielsweise in KBE(ml), bestimmt werden kann. Die achte V8, neunte V9 und zehnte V10 Ventilschaltungen können dabei in durchleitende oder blockierende Stellung gebracht werden, um die Probenentnahme zu unterstützen. Für eine Probenentnahme an der Probenentnahmestelle P1 wird die achte Ventilschaltung V8 und die zehnte Ventilschaltung in eine blockierende Stellung gebracht und die neunte Ventilschaltung V9 wir in eine durchleitende Stellung gebracht.

In einer weiter gebildeten Ausführungsform der erfindungsgemäßen Vorrichtung zur Aufbereitung von Flüssigkeiten, insbesondere Wasserzeigt Fig. 1 weiterhin eine erste Fluidabzweigung 111a in der Fluidleitung 111 zur Ableitung von Flüssigkeit aus dem Retentatraum F_{1R} des ersten Hohlfasermembranfilters F1, eine von der Fluidverzweigung 111a erste abzweigende Fluidleitung 113, eine im Eingriff mit dieser Fluidleitung stehenden Ventilschaltung V2 und in Flüssigkeitsverbindung mit der Fluidleitung 113 eine Probenentnahmestelle P2, sowie eine elfte Ventilschaltung V11. Zur Aufbereitung der Flüssigkeit, werden die Ventilschaltungen V1 und V2 in eine blockierende Stellung gebracht. In den Retentatraum F_{1R} des ersten Hohlfasermembranfilters F1 wird Flüssigkeit, insbesondere Wasser, eingeleitet und über die Membranwandung F_{1M} in den Filtratraum F_{1F} des ersten Hohlfasermembranfilters F1 überführt. Die im Filtratraum F_{1F} anfallende Flüssigkeit wird über Fluidanschlüsse 103a/b, 120 und Fluidleitung 112 in den Retentatraum F_{2R} des zweiten Hohlfasermembranfilters überführt, um gegebenenfalls in einer weiteren Filtration im "Tangential-Flow Verfahren" eine aufbereitete Flüssigkeit, insbesondere Wasser zur Herstellung einer Dialysierflüssigkeit, bereitzustellen.

Der Retentatraum F_{1R} des ersten Hohlfasermembranfilters kann auf Kontamination geprüft werden, indem die Stellung der Ventilschaltung V2 in eine durchleitende Stellung gebracht wird. Die angereicherten Kontaminanten im Retentatraum F_{1R} des ersten Hohlfasermembranfilters F1 können auf diese Weise über die Fluidleitung 113 abgeleitet werden. An der Probenentnahmestelle P2 können Proben entnommen werde und der Analyse zugeführt werden. Das elfte Ventil V1 wird zur Probenentnahme auf eine durchleitende Stellung gebracht.

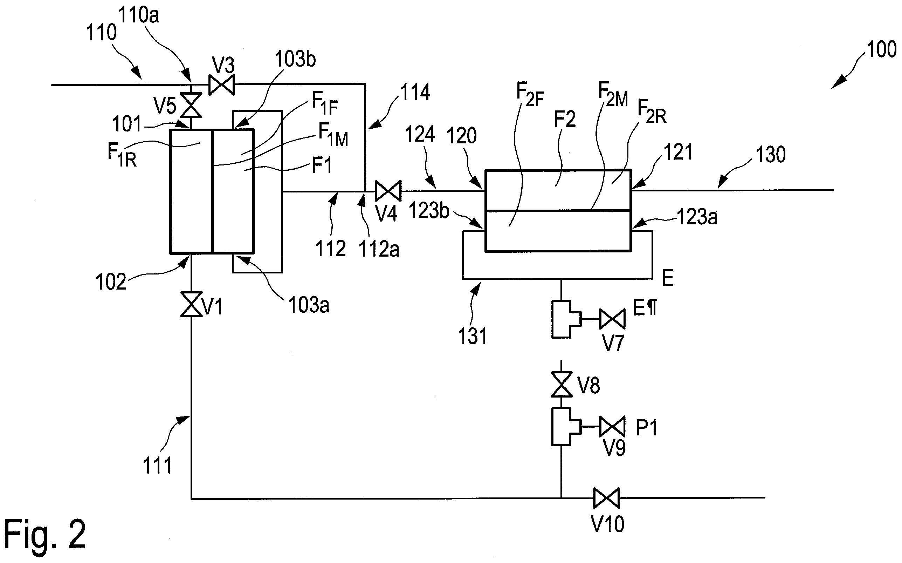

Fig.2 zeigt eine weitere schematische Abbildung einer weiteren Ausführungsform der erfindungsgemäßen Vorrichtung 100 zur Aufbereitung von Flüssigkeiten, insbesondere Wasser. Zusätzlich zu Fig. 1 zeigt Fig. 2 eine fünfte Ventilschaltung im Eingriff mit der Fluidleitung 110 zum Zuführen von Flüssigkeit, insbesondere Wasser in den Retentatraum F_{1R} des ersten Hohlfasermembranfilters, eine zweite Fluidverzweigung 110a, eine dritte Fluidverzweigung 112a in der Fluidleitung 112 zur Ableitung von Flüssigkeit, insbesondere Wasser aus dem Filtratraum F_{1F} des ersten Hohlfasermembranfilters F1, eine Fluidleitung 114 in Flüssigkeitsverbindung mit der zweiten Fluidabzweigung 110a und der dritten Fluidverzweigung 112a, sowie eine dritte Ventilschaltung V3 im Eingriff mit der Fluidleitung 114, die in Flüssigkeitsverbindung mit der zweiten Fluidverzweigung 110a und der dritten Fluidverzweigung 112a steht.

Die Aufbereitung von Flüssigkeit erfolgt durch Einleiten von Flüssigkeit, insbesondere Wasser, in den Retentatraum F_{1R} des ersten Hohlfasermembranfilters F1, wobei die fünfte Ventilschaltung V5 in eine durchleitende Stellung gebracht ist, und die dritte V3 und die erste V1 Ventilschaltungen in eine blockierende Stellung gebracht sind. Flüssigkeit, insbesondere Wasser, wird über die Fluidleitung 110 und den Fluidanschluss 101 in den Retentatraum F_{1R} des ersten Hohlfasermembranfilters eingeleitet. Das Einleiten von Wasser kann über Pumpenmittel erfolgen, die in der vorliegenden Fig. 2 nicht abgebildet sind. Die Flüssigkeit wird über die Membranwandung F_{1M} in den Filtratraum F_{1F} des ersten Hohlfasermembranfilters F1 überführt und über die Fluidanschlüsse 103a/b, 120 und Fluidleitung 112 in den Retentatraum F_{2R} des zweiten Hohlfasermembranfilters F2 übergeleitet. Die vierte Ventilschaltung befindet sich in einer durchleitenden Ventilstellung. Gegebenenfalls wird in einer weiteren Filtration am zweiten Hohlfasermembranfilter F_{2R} im "Tangential-Flow Verfahren" eine aufbereitete Flüssigkeit, insbesondere Wasser für die Herstellung einer Dialysierflüssigkeit, bereitgestellt.

Gemäß der Darstellung in Fig. 2 kann die erfindungsgemäße Vorrichtung in einem "Vorspülverfahren" auf Kontamination des Retentatraumes F_{1R} untersucht werden. Entsprechend werden die fünfte und die erste Ventilschaltungen V5 und V1 in eine durchleitende Ventilstellung gebracht und die dritte und vierte Ventilschaltung V3 und V 4 in eine blockierende Ventilstellung gebracht. Angereicherte Kontaminanten können in dieser Anordnung der Ventilschaltungen und Ventilstellungen aus dem Retentatraum F_{1R} des ersten Hohlfasermembranfilters abgeleitet werden. An der Probenentnahmestelle P1 können Proben entnommen werden und auf Kontamination analysiert werden.

Zusätzlich kann gemäß der Darstellung in Fig. 2 in einem "Rückspülverfahren" auf Kontamination des Retentatraumes F_{1R} des ersten Hohlfasermembranfilters untersucht werden. Hierzu werden die fünfte Ventilschaltung V5 und die vierte Ventilschaltung V4 in eine blockierende Ventilstellung gebracht und die dritte Ventilschaltung V3 und die erste Ventilschaltung V1 in eine durchleitende Ventilstellung gebracht. Flüssigkeit, insbesondere Wasser, wird anschließend über die Fluidleitungen 110, 114, 112, und die Fluidanschlüsse 103a/b in den Filtratraum F_{1F} des ersten Hohlfasermembranfilters F1 eingeleitet und über die Membranwandung F_{1M} in den Retentatraum F_{1R} des ersten Hohlfasermembranfilters F1 überführt. Die im Retentatraum F_{1R} anfallende Flüssigkeit, insbesondere Wasser, wird mit den im Retentatraum F_{1R} angereicherten Kontaminanten über die Fluidleitung 111 abgeleitet. An der Probenentnahmestelle P1 können Proben entnommen werden und der Analyse zugeführt werden, so dass Rückschlüsse auf die Kontamination des Retentatraumes F_{1R} des ersten Hohlfasermembranfilters F1 geschlossen werden können. Ein kombiniertes Vor- und Rückspülverfahren ist bevorzugt, um angereicherte Kontaminanten im Retentatraum F_{1R} des ersten Hohlfasermembranfilters F1 untersuchen zu können. Insbesondere können durch das Rückspülverfahren Kontaminanten, die im Retentatraum F_{1R} an der Membranwandung F_{1M} anhaften freigespült werden und der Analyse zugänglich gemacht werden.

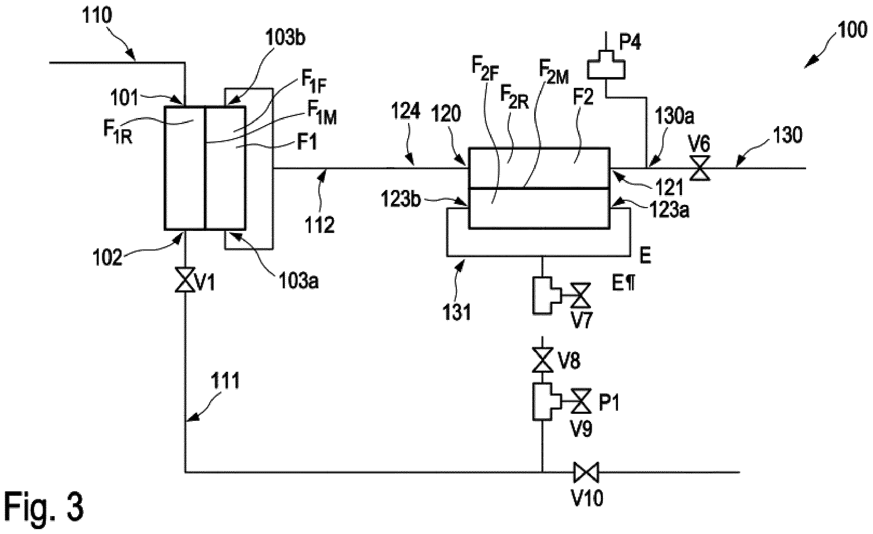

Fig. 3 zeigt eine weitere schematische Abbildung einer weiteren Ausführungsform der erfindungsgemäßen Vorrichtung 100 zur Aufbereitung von Flüssigkeiten, insbesondere Wasser. Zusätzlich zu Fig. 1 zeigt Fig. 3 eine vierte Fluidverzweigung 130a, eine sechste Ventilschaltung V6 im Eingriff mit der Fluidleitung 130 zur Ableitung von Flüssigkeit aus dem Retentatraum F_{2R} des zweiten Hohlfasermembranfilters F2, eine zweite abzweigende Fluidleitung 131, die die vierte Fluidverzweigung 130a mit einer vierten Probenentnahmestelle P4 in Fluidverbindung bringt. Die Probenentnahmestelle P4 dient zur Probenentnahme von Flüssigkeit, insbesondere Wasser, die aus dem Retentatraum F_{2R} des zweiten Hohlfasermembranfilters F2 abgeleitete wird, um durch Analyse Kontaminanten feststellen zu können.

Zur Aufbereitung von einer Flüssigkeit, insbesondere Wasser, wird Flüssigkeit, insbesondere Wasser über die Fluidleitung 110 und den Fluidanschluss 101 in den Retentatraum F_{1R} des ersten Hohlfasermembranfilters eingeleitet wird. Die Stellung der ersten Ventilschaltung ist auf eine blockierende Ventilstellung eingestellt. Das Einleiten von Flüssigkeit, insbesondere Wasser in den Retentatraum F_{1R} kann über Pumpenmittel erfolgen, die in der vorliegenden Fig. 3 nicht abgebildet sind. Die Flüssigkeit wird über die Membranwandung F_{1M} in den Filtratraum F_{1F} des ersten Hohlfasermembranfilters F1 überführt und über die Fluidanschlüsse 103a/b, 120 und Fluidleitung 112 in den Retentatraum F_{2R} des zweiten Hohlfasermembranfilters F2 übergeleitet. In einem weiteren Schritt wird Flüssigkeit, insbesondere Wasser im "Tangential-Flow Verfahren" im zweiten Hohlfasermembranfilter F2 verarbeitet. Dabei wird Flüssigkeit, insbesondere Wasser, wie vorab beschrieben über die Fluidleitung 112 und den Fluidanschluss 120 in den Retentatraum F_{2R} des zweiten Hohlfasermembranfilters F2 eingeleitet, wobei die sechste Ventilschaltung V6 in eine durchleitende Ventilstellung gebracht ist. Ein Teil der in den Retentatraum F_{2R} des zweiten Hohlfasermembranfilters eingeleiteten Flüssigkeit, insbesondere Wasser, tritt gemäß des "Tangential-Flow" Verfahrens in den Filtratraum F_{2F} des zweiten Hohlfasermembranfilters über, und kann über die Fluidanschlüsse 123a/b und Fluidleitung 131 abgeleitet werden und über die Entnahmestelle E entnommen werden, z.B. zur Herstellung einer Substitutionsflüssigkeit oder zur Entnahme von Proben für die Analyse auf Kontamination, sofern die siebte Ventilschaltung V7 in einer durchleitenden Ventilstellung gebracht ist. Ein weiterer Teil der Flüssigkeit, insbesondere Wasser, das im Retentatraum F_{2R} des zweiten Hohlfasermembranfilters eingeleitet wurde wird über den Fluidanschluss 121 und Fluidleitung 130 abgeleitet und kann weiterverarbeitet werden, z.B. für die Herstellung einer Dialysierflüssigkeit.

Die aus dem Retentatraum F_{2R} des zweiten Hohlfasermembranfilters abgeleitete Flüssigkeit, insbesondere Wasser, kann auf Kontamination untersucht werden, indem die sechste Ventilschaltung V6 in eine blockierende Ventilstellung gebracht wird und Proben an der Probenentnahmestelle P4 entnommen werden und auf Kontamination analysiert werden. Die Vorrichtung ermöglicht damit eine Analyse der über die "Dead-End" Filtration am ersten Hohlfasermembranfilter F1 gewonnenen Flüssigkeit, insbesondere Wasser,

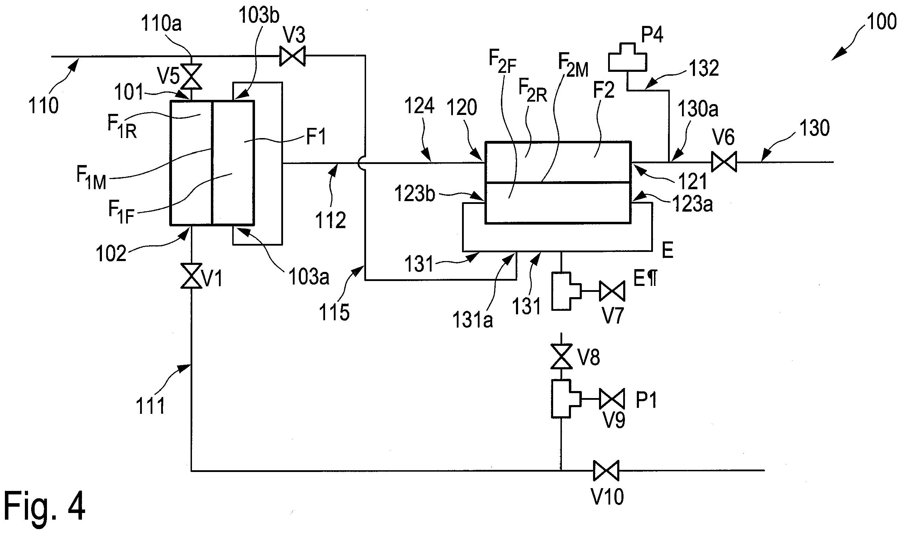

Fig. 4 zeigt eine weitere schematische Abbildung einer weiteren Ausführungsform der erfindungsgemäßen Vorrichtung 100 zur Aufbereitung von Flüssigkeiten, insbesondere Wasser. Fig. 4 zeigt im Gegensatz zu Fig.2 keine dritte Fluidverzweigung 112 in der Fluidleitung zur Ableitung von Flüssigkeit aus dem Filtratraum F_{1F} des ersten Hohlfasermembranfilters F1. Weiterhin weist die Vorrichtung nach Fig. 4 keine Fluidleitung 114 auf, die die zweite Fluidverzweigung 110a mit der dritten Fluidverzweigung 112a der Vorrichtung nach Fig. 2 in Flüssigkeitsverbindung bringt. Die Vorrichtung nach Fig. 4 ist gegenüber der Ausführung nach Fig. 2 dadurch gekennzeichnet, dass in der Fluidleitung 131 zur Ableitung von Flüssigkeit, insbesondere Wasser, aus dem Filtratraum F_{2F} des zweiten Hohlfasermembranfilters F2 eine fünfte Fluidverzweigung131a angeordnet ist. Gemäß der Anordnung nach Fig. 4 ist die Vorrichtung weiter dadurch gekennzeichnet, dass eine Fluidleitung 115 die zweite Fluidverzweigung 110a mit der fünften Fluidverzweigung 131a in Flüssigkeitsverbindung bringt.

Gemäß den vorherigen Beschreibungen dient die Vorrichtung nach Fig. 4 zur Wasseraufbereitung von Flüssigkeit, insbesondere Wasser, wobei die Aufbereitung am ersten Hohlfasermembranfilter F1 nach einer "Dead-End" Filtration verläuft und die Aufbereitung am zweiten Hohlfasermembranfilter nach dem "Tangential-Flow" Verfahren erfolgt. Durch Einstellen der fünften und der ersten Ventilschaltung in eine durchleitende Ventilstellung und Einstellen der Ventilschaltung V3 in eine Blockierende Ventilstellung können über die Probenentnahmestelle P1 Proben entnommen werden und auf Kontamination analysiert werden, so dass Rückschlüsse auf die Kontamination des Retentatraums F_{1R} des ersten Hohlfasermembranfilters F1 gemacht werden können.

Durch Einstellen der ersten Ventilschaltung V1 und der sechsten Ventilschaltung V6 in eine blockierende Ventilstellung und Einstellen der fünften Ventilschaltung V5 in eine durchleitende Ventilstellung können über die Probeentnahmestelle P4 Proben entnommen werden und auf Kontamination analysiert werden, so dass Rückschlüsse über die Kontamination des Filtratraums F_{1F} des ersten Hohlfasermembranfilters der Fluidleitung 112 und des Retentatraums F_{2R} des zweiten Hohlfasermembranfilters gemacht werden können.

Durch Einstellen der fünften Ventilschaltung V5 und der ersten Ventilschaltung V1 in eine blockierende Ventilstellung und Einstellen der dritten Ventilschaltung V3 in eine durchleitende Ventilstellung wird Flüssigkeit über die Fluidleitung 110 zur Zuführung von Flüssigkeit zum Retentatraum F_{1R} des ersten Hohlfasermembranfilters und die Fluidleitung 115, die die zweite Fluidverzweigung 110a mit der fünften Fluidverzweigung 131a in Flüssigkeitsverbindung bringt über die Fluidleitung 131 in den Filtratraum F_{2F} eingeleitet und über die Membranwandung F_{2M} des zweiten Hohlfasermembranfilters F2 in den Retentatraum F_{2R} des zweiten Hohlfasermembranfilters überführt. Die Filtration von Flüssigkeit, insbesondere Wasser, am zweiten Hohlfasermembranfilter F2 erfolgt in dieser Ventilanordnung und Einstellung der Ventilstellung vom Filtratraum in den Retentatraum des zweiten Hohlfasermembranfilter nach dem Prinzip einer Rückspülung. Durch Rückspülung des zweiten Hohlfasermembranfilters F2 können Kontaminanten, die an der Membranwandung F_{2M} des zweiten Hohlfasermembranfilters anhaften freigespült werden. Über die Probenentnahmestelle P4 können Proben entnommen werden und auf Kontamination analysiert werden. Die Analyse lässt Rückschlüsse auf die Kontamination des zweiten Hohlfasermembranfilters zu.

## Patentansprüche

1. Vorrichtung (100) zur Aufbereitung einer Flüssigkeit, insbesondere zur Aufbereitung von Wasser für die Dialyse,
aufweisend einen ersten Hohlfasermembranfilter (F1), wobei der erste Hohlfasermembranfilter weiter aufweist,
eine Vielzahl von Hohlfasermembranen, die einen Retentatraum (F_{1R}) und einem Filtratraum (F_{1F}) bilden, wobei der Retentatraum (F_{1R}) und der Filtratraum (F_{1F}) über die semipermeablen Membranwandungen (F_{1M}) der Hohlfasermembranen voneinander getrennt sind,
einen Fluidanschluss (101) zur Zuführung von Flüssigkeit, insbesondere Wasser in den Retentatraum,
einen Fluidanschluss zur Ableitung (102) von Flüssigkeit, insbesondere Wasser, aus dem Retentatraum,
wenigstens einen Fluidanschluss (103a/ 103b) zur Ableitung von Flüssigkeit, insbesondere Wasser, aus dem Filtratraum,
wobei die Vorrichtung (100) weiter aufweist,
eine Fluidleitung (110) in Flüssigkeitsverbindung mit dem Fluidanschluss zur Zuführung von Flüssigkeit, insbesondere Wasser, in den Retentatraum (F_{1R}) des ersten Hohlfasermembranfilters (F1),
eine Fluidleitung (111) in Flüssigkeitsverbindung mit dem Fluidanschluss (102) zur Ableitung von Flüssigkeit, insbesondere Wasser, aus dem Retentatraum (F_{1R}) des ersten Hohlfasermembranfilters (F1),
eine Fluidleitung (112) in Flüssigkeitsverbindung mit dem Fluidanschluss (102) zur Ableitung von Flüssigkeit, insbesondere Wasser, aus dem Filtratraum (F_{1F}) des ersten Hohlfasermembranfilters (F1),
eine erste Ventilschaltung (V1) im Eingriff mit der Fluidleitung (111) zum Ableiten von Flüssigkeit, insbesondere Wasser, aus dem Retentatraum, wobei die Ventilschaltung wenigstens zwei Ventilstellungen umfasst, wonach der Durchfluss von Flüssigkeit durch die Fluidleitung (111) zum Ableiten von Flüssigkeit aus dem Retentatraum, blockiert werden kann oder durchgeleitet werden kann,
optional weiter aufweisend
eine erste Fluidverzweigung (111 a) in der Fluidleitung (111) zur Ableitung von Flüssigkeit, insbesondere Wasser, aus dem Retentatraum (F_{1R}) des ersten Hohlfasermembranfilters (F1), in Flüssigkeitsverbindung mit einer ersten abzweigende Fluidleitung (113), eine zweite Ventilschaltung (V2) im Eingriff mit der ersten abzweigenden Fluidleitung (113), wobei die zweite Ventilschaltung (V2) wenigstens zwei Ventilstellungen umfasst, wonach der Durchfluss von Flüssigkeit, insbesondere Wasser durch die erste abzweigende Fluidleitung (113) blockiert werden kann oder durchgeleitet werden kann,
**dadurch gekennzeichnet, dass**
stromabwärts der ersten Ventilschaltung (V1) in Flüssigkeitsverbindung mit der Fluidleitung (111) zum Ableiten von Flüssigkeit, insbesondere Wasser, aus dem Retentatraum (F_{1R}), eine Probeentnahmestelle (P1) angeordnet ist
und/ oder, dass
stromab der zweiten Ventilschaltung (V2) in Flüssigkeitsverbindung zu der ersten abzweigenden Fluidleitung (113) eine zweite Probenentnahmestelle (P2) angeordnet ist.

2. Vorrichtung nach Anspruch 1, **dadurch gekennzeichnet, dass** in der Fluidleitung (110) zur Zuführung von Flüssigkeit, insbesondere Wasser, in den Retentatraum (F_{1R}) stromaufwärts des ersten Hohlfasermembranfilters (F1) eine zweite Fluidabzweigung (110a) angeordnet ist,
weiter **gekennzeichnet dadurch, dass** in der Fluidleitung (112) zur Ableitung von Flüssigkeit, insbesondere Wasser, aus dem Filtratraum (F_{1F}) des ersten Hohlfasermembranfilters (F1) eine dritte Fluidabzweigung (112a) angeordnet ist,
weiter **gekennzeichnet dadurch, dass** die zweite Fluidabzweigung (110a) an der Fluidleitung (110) zur Zuführung von Flüssigkeit, insbesondere Wasser, in den Retentatraum (F_{1F}) des ersten Hohlfasermembranfilters (F1) und die dritte Fluidabzweigung (112a) an der Fluidleitung (112) zur Ableitung von Flüssigkeit, insbesondere Wasser aus dem Filtratraum (F_{1F}) des ersten Hohlfasermembranfilters (F1), über eine Fluidleitung (114) flüssigkeitsverbindend verbunden sind,
weiter **gekennzeichnet dadurch, dass** im Eingriff mit der Fluidleitung (114) zwischen der zweiten Fluidabzweigung (110a) und der dritten Fluidabzweigung (112a) eine dritte Ventilschaltung (V3) angeordnet ist, wobei die dritte Ventilschaltung (V3) wenigstens zwei Ventilstellungen umfasst, wonach der der Durchfluss von Flüssigkeit, insbesondere Wasser, durch die Fluidleitung(114) zwischen der zweiten Fluidabzweigung (110a) und der dritten Fluidabzweigung (112a) blockiert werden kann oder durchgeleitet werden kann,
weiter **gekennzeichnet dadurch, dass** stromabwärts der dritten Fluidabzweigung (112a) im Eingriff mit der Fluidleitung (112) zur Ableitung von Flüssigkeit, insbesondere Wasser, aus dem Filtratraum (F_{1F}) eine vierte Ventilschaltung (V4) angeordnet ist, wobei die vierte Ventilschaltung (V4) wenigstens zwei Ventilstellungen umfasst, wonach der Durchfluss von Flüssigkeit, insbesondere Wasser, durch die Fluidleitung (112) zur Ableitung von Flüssigkeit, insbesondere Wasser aus dem Filtratraum blockiert werden kann oder durchgeleitet werden kann.

3. Vorrichtung nach Anspruch 2, **dadurch gekennzeichnet, dass** stromaufwärts des Hohlfasermembranfilters (F1) zwischen der zweiten Fluidabzweigung (110a) an der Fluidleitung (110) zur Zuführung von Flüssigkeit, insbesondere Wasser in den Retentatraum (F_{1R}) des ersten Hohlfasermembranfilters und dem Hohlfasermembranfilter (F1) im Eingriff mit der Fluidleitung (110) zur Zuführung von Flüssigkeit, insbesondere Wasser in den Retentatraum (F_{1R}) des ersten Hohlfasermembranfilters (F1) eine fünfte Ventilschaltung (V5) angeordnet ist, wobei die fünfte Ventilschaltung (V5) wenigstens zwei Ventilstellungen umfasst, wonach der Durchfluss von Flüssigkeit, insbesondere Wasser, durch die Fluidleitung (110) zum Zuführen von Flüssigkeit, insbesondere Wasser in den Retentatraum (F_{1R}) des ersten Hohlfasermembranfilters (F1), blockiert werden kann oder durchgeleitet werden kann.

4. Vorrichtung nach mindestens einem der Ansprüche 1 bis 3, **dadurch gekennzeichnet, dass** die Vorrichtung weiter aufweist
einen zweiten Hohlfasermembranfilter (F2), wobei der zweite Hohlfasermembranfilter weiter aufweist,
eine Vielzahl von Hohlfasermembranen, mit einem Retentatraum (F_{2R}) und einem Filtratraum (F_{1F}), die über semipermeable Membranwandungen (F_{2M}) der Hohlfasermembranen voneinander getrennt sind,
einen Fluidanschluss (120) zur Zuführung von Flüssigkeit, insbesondere Wasser in den Retentatraum (F_{2R}) des zweiten Hohlfasermembranfilters,
einen Fluidanschluss (121) zur Ableitung von Flüssigkeit, insbesondere Wasser aus dem Retentatraum (F_{2R}) des zweiten Hohlfasermembranfilters,
wenigstens einen Fluidanschluss (123a/ 123b) zum Ableiten von Flüssigkeit, insbesondere Wasser aus dem Filtratraum des zweiten Hohlfasermembranfilters,
eine Fluidleitung (124) in Flüssigkeitsverbindung mit dem Fluidanschluss (120) zur Zuführung von Flüssigkeit, insbesondere Wasser in den Retentatraum (F_{2R}) des zweiten Hohlfasermembranfilters,
und wobei die Vorrichtung (100) weiter aufweist,
eine Fluidleitung (130) in Flüssigkeitsverbindung mit dem Fluidanschluss 121 zum Ableiten von Flüssigkeit, insbesondere Wasser aus dem Retentatraum (F_{2R}) des zweiten Hohlfasermembranfilters,
eine Fluidleitung (131) in Flüssigkeitsverbindung mit dem wenigstens einen Fluidanschluss (123a/ 123b) zum Ableiten von Flüssigkeit, insbesondere Wasser aus dem Filtratraum (F_{2F}) des zweiten Hohlfasermembranfilters (F2),wobei die Vorrichtung weiter durch **dadurch gekennzeichnet ist, dass** die Fluidleitung (124) in Flüssigkeitsverbindung mit dem Fluidanschluss (120) zur Zuführung von Flüssigkeit, insbesondere Wasser in den Retentatraum (F_{2R}) des zweiten Hohlfasermembranfilters (F2) mit der Fluidleitung (112) zur Ableitung von Flüssigkeit, insbesondere Wasser, aus dem Filtratraum (F_{1F}) des ersten Hohlfasermembranfilters (F1) flüssigkeitsverbindend verbunden ist.

5. Vorrichtung nach Anspruch 4, **dadurch gekennzeichnet, dass** in der Fluidleitung (130) zwischen Fluidanschluss (121) zur Ableitung von Flüssigkeit, insbesondere Wasser aus dem Retentatraum (F_{2R}) des zweiten Hohlfasermembranfilters (F2) eine vierte Fluidabzweigung (130a) angeordnet ist, von der eine zweite abzweigende Fluidleitung (132) in Flüssigkeitsverbindung zur Fluidverzweigung (130a) abzweigt, weiterhin **dadurch gekennzeichnet, dass** stromab der vierten Fluidabzweigung (130a) eine sechste Ventilschaltung (V6) im Eingriff mit der der Fluidleitung (130) zum Ableiten von Flüssigkeit, insbesondere Wasser, aus dem Retentatraum (F_{2F}) des zweiten Hohlfasermembranfilters (F2) angeordnet ist, wobei die sechste Ventilschaltung (V6) wenigstens zwei Ventilstellungen umfasst, wonach der der Durchfluss von Flüssigkeit, insbesondere Wasser, durch die Fluidleitung (130) zum Ableiten von Flüssigkeit, insbesondere Wasser, aus dem Retentatraum (F_{2R}) des zweiten Hohlfasermembranfilters (F2), blockiert werden kann oder durchgeleitet werden kann;
weiterhin **dadurch gekennzeichnet, dass** die zweite abzweigende Fluidleitung (132) mit einer vierten Probeentnahmestelle (P4) in Flüssigkeitsverbindung steht.

6. Vorrichtung nach Anspruch 1, **dadurch gekennzeichnet, dass** die Vorrichtung weiter wenigstens einen zweiten Hohlfasermembranfilter (F2) aufweist,
wobei der zweite Hohlfasermembranfilter aufweist,
eine Vielzahl von Hohlfasermembranen, die einen Retentatraum (F_{2R}) und einen Filtratraum (F_{2F}) bilden, die über eine semipermeable Membranwandung (F_{2M}) der Hohlfasermembranen voneinander getrennt sind,
einen Fluidanschluss (120) zur Zuführung von Flüssigkeit, insbesondere Wasser, in den Retentatraum (F_{2R}) des zweiten Hohlfasermembranfilters,
einen Fluidanschluss (121) zur Ableitung von Flüssigkeit, insbesondere Wasser, aus dem Retentatraum (F_{2R}) des zweiten Hohlfasermembranfilters,
wenigstens einen Fluidanschluss (123a/ 123b) zum Ableiten von Flüssigkeit, insbesondere Wasser, aus dem Filtratraum (F_{2F}) des zweiten Hohlfasermembranfilters,
und wobei die Vorrichtung (100) weitere aufweist,
wenigstens eine Fluidleitung (112) zur Zuführung von Flüssigkeit, insbesondere Wasser in den Retentatraum (F_{2F}) des zweiten Hohlfasermembranfilters (F2),
wenigstens eine Fluidleitung (131) in Flüssigkeitsverbindung mit dem wenigstens einen Fluidanschluss (123a/ 123b) zum Ableiten von Flüssigkeit, insbesondere Wasser, aus dem Filtratraum (F_{2F}) des zweiten Hohlfasermembranfilters,
wobei die Vorrichtung (100) weiter **dadurch gekennzeichnet ist, dass** in der Fluidleitung (110) zur Zuführung von Flüssigkeit, insbesondere Wasser, in den Retentatraum (F_{1R}) des ersten Hohlfasermembranfilters (F1) stromaufwärts des ersten Hohlfasermembranfilters (F1) eine zweite Fluidverzweigung (110a) angeordnet ist, weiter **dadurch gekennzeichnet, dass** die Vorrichtung (100) an der Fluidleitung (131) zur Ableitung von Flüssigkeit, insbesondere Wasser, aus dem Filtratraum (F_{2F}) des zweiten Hohlfasermembranfilters (F2) eine fünfte Fluidverzweigung (131a) aufweist, wobei eine vierte Probeentnahmestelle (P4) über eine vierte Verzweigungsstelle (130a) in der Fluidleitung (130) zur Ableitung von Flüssigkeit, insbesondere Wasser, aus dem Retentatraum (F_{2R}) des zweiten Hohlfasermembranfilters (F2) in Flüssigkeitsverbindung angeordnet ist,
weiter **dadurch gekennzeichnet, dass** eine Fluidleitung (115) in Flüssigkeitsverbindung mit der zweiten Fluidverzweigung (110a) an der Fluidleitung (110) zur Zuführung von Flüssigkeit, insbesondere Wasser, in den Retentatraum (F_{1R}) des ersten Hohlfasermembranfilters (F1) und der fünften Fluidverzweigung (131a) an der Fluidleitung (131) zur Ableitung von Flüssigkeit, insbesondere Wasser, aus dem Filtratraum (F_{2F}) des zweiten Hohlfasermembranfilters (F2) angeordnet ist, weiter **dadurch gekennzeichnet, dass** im Eingriff mit der Fluidleitung (115) zwischen der zweiten Fluidverzweigung (110a) an der Fluidleitung (110) zur Zuführung von Flüssigkeiten, insbesondere Wasser, in den Retentatraum (F_{1R}) ersten Hohlfasermembranfilters (F1) und der fünften Fluidverzweigung (131a) an der Fluidleitung (131) zur Ableitung von Flüssigkeit, insbesondere Wasser aus dem Filtratraum (F_{2F}) des zweiten Hohlfasermembranfilters (F2) eine dritte Ventilschaltung (V3) angeordnet ist, wobei die dritte Ventilschaltung (V3) wenigstens zwei Ventilstellungen umfasst, wonach der der Durchfluss von Flüssigkeit, insbesondere Wasser, durch die Fluidleitung (115) zwischen der zweiten Fluidabzweigung und der fünften Fluidabzweigung blockiert werden kann oder durchgeleitet werden kann,
weiter **dadurch gekennzeichnet, dass** der Fluidanschluss (120) zum Zuführen von Flüssigkeit, insbesondere Wasser, in den Retentatraum (F_{2R}) des zweiten Hohlfasermembranfilters (F2) über eine Fluidleitung (112) mit dem wenigstens einen Fluidanschluss (103a/ 103b) zum Ableiten von Flüssigkeit, insbesondere Wasser, aus dem Filtratraum (F_{1F}) des ersten Hohlfasermembranfilters (F1) in Flüssigkeitsverbindung steht.
weiter **dadurch gekennzeichnet, dass** stromaufwärts des Filters (F1) zwischen der zweiten Fluidverzweigung (110a) im Eingriff mit der Fluidleitung (110) zur Zuführung von Flüssigkeit, insbesondere Wasser, in den Retentatraum (F_{1R}) und dem ersten Hohlfasermembranfilter Filter (F1) eine fünfte Ventilschaltung (V5) angeordnet ist, wobei die fünfte Ventilschaltung (V5) wenigstens zwei Ventilstellungen umfasst, wonach der der Durchfluss von Flüssigkeit, insbesondere Wasser, durch die Fluidleitung (110) zur Zuführung von Flüssigkeit, insbesondere Wasser, in den Retentatraum blockiert werden kann oder durchgeleitet werden kann.

7. Vorrichtung nach mindestens einem der vorhergehenden Ansprüche , wobei
die Vorrichtung eine Analysevorrichtungen aufweist, mit der an einer Probenentnahmestellen einer gewonnenen Probe ein Analysewert bezüglich der Konzentration von Kontaminanten in einer jeweiligen Probe ermittelt werden kann, wobei
die Vorrichtung weiterhin Mittel zur Bestimmung des Volumens der vom Retentatraum in den Filtratraum des ersten Hohlfasermembranfilters überführten Flüssigkeit, insbesondere Wasser, aufweist, wobei
die Vorrichtung weiterhin Mittel zur Bestimmung des Volumens an Flüssigkeit, insbesondere Wasser, mit der die Kontaminanten zu der Probenentnahmestelle gespült werden, aufweist, wobei die Vorrichtung weiterhin eine elektronische Auswerteeinheit aufweist, **dadurch gekennzeichnet, dass**
die Auswerteeinheit so konfiguriert ist, dass sie aus dem Analysewert bezüglich der Konzentration von Kontaminanten in einer jeweiligen Probe, dem Volumen der vom Retentatraum in den Filtratraum des ersten Hohlfasermembranfilters überführten Flüssigkeit, insbesondere Wasser, und dem Volumen an Flüssigkeit, insbesondere Wasser, mit der die Kontaminanten zu der Probenentnahmestelle gespült werden, einen Umrechnungsfaktor ermittelt, mit dem ein Wert für eine Kontamination der für die Vorrichtung bereitgestellten Flüssigkeit, insbesondere Wasser, ermittelt werden.

8. Verfahren zur Überwachung der Aufbereitung von Flüssigkeiten, insbesondere zur Aufbereitung von Wasser für die Dialyse, aufweisend die Schritte
Bereitstellen einer Vorrichtung (100) nach Anspruch 1
Einstellen der ersten Ventilschaltung (V1) und der gegebenenfalls zweiten Ventilschaltung (V2) in eine blockierende Stellung,
Einleiten von Flüssigkeit, insbesondere Wasser, in den Retentatraum (F_{1R}) des ersten Hohlfasermembranfilters (F1),
Filtrieren der Flüssigkeit, insbesondere Wasser über die Membranwandung (F_{1M}) der Hohlfasermembranen von dem Retentatraum (F_{1R}) in den Filtratraum (F_{1F}) des ersten Hohlfasermembranfilters (F1) und Auffangen der Flüssigkeit, insbesondere Wasser, im Filtratraum (F_{1F}), wobei bei der Filtration Kontaminanten im Rohwasser von der Hohlfasermembran zurückgehalten werden,
Ableiten der Flüssigkeit, insbesondere des Wassers, aus dem Filtratraum (F_{1F}),
Einstellen der ersten Ventilschaltung (V1) in eine durchleitende Stellung, nachdem eine vorherbestimmte Menge an Flüssigkeit, insbesondere Wasser durch Filtration in den Filtratraum überführt wurde,
wobei die gegebenenfalls zweite Ventilschaltung in einer blockierenden Ventilstellung verbleibt,
weiteres Einleiten von Flüssigkeit, insbesondere Wasser, in den Retentatraum (F_{1R}) des ersten Hohlfasermembranfilters (F1),
**dadurch gekennzeichnet, dass** an der ersten Probenentnahmestelle (P1) stromabwärts der ersten Ventilschaltung (V1) Proben entnommen werden und die Proben auf Kontamination analysiert werden,
oder gegebenenfalls
Einstellen der ersten Ventilschaltung (V1) in eine blockierende Ventilstellung, und Einstellen der zweiten Ventilschaltung in eine durchleitende Ventilstellung nachdem eine vorherbestimmte Menge an Flüssigkeit, insbesondere Wasser, durch Filtration in den Filtratraum überführt wurde,
weiteres Einleiten von Flüssigkeit, insbesondere Wasser, in den Retentatraum (F_{1R}) des ersten Hohlfasermembranfilters (F1),
**dadurch gekennzeichnet, dass** an der zweiten Probenentnahmestelle (P2) stromabwärts der ersten Fluidverzweigung (11 1a) Proben entnommen werden und die Proben auf Kontamination analysiert werden.

9. Verfahren nach Anspruch 8, **dadurch gekennzeichnet, dass** die vorherbestimmte Menge an Flüssigkeit, insbesondere Wasser, die durch Filtration in den Filtratraum (F_{1F}) des ersten Hohlfasermembranfilters überführt wurde mindestens 20 l, oder mehr, bevorzugt mindestens 100 I oder mehr, weiter bevorzugt mindestens 200 l oder mehr beträgt.

10. Verfahren zur Überwachung der Aufbereitung von Flüssigkeiten, insbesondere Wasser für die Dialyse, aufweisend die Schritte,
Bereitstellen einer Vorrichtung (100) nach Anspruch 2
Einstellen der ersten Ventilschaltung (V1) in eine blockierende Ventilstellung,
Einstellen der dritten Ventilschaltung (V3) in eine blockierende Ventilstellung,
Einstellen der vierten Ventilschaltung (V4) in eine durchleitende Ventilstellung,
Einleiten von Flüssigkeit, insbesondere Wasser, in den Retentatraum (F_{1R}) des ersten Hohlfasermembranfilters (F1),
Filtrieren der Flüssigkeit, insbesondere Wasser, über die Membranwandung (F_{1M}) der Hohlfasermembranen des ersten Hohlfasermembranfilters (F1), wobei bei der Filtration Kontaminanten der Flüssigkeit, insbesondere Wasser, von der Membranwandung (F_{1M}) der Hohlfasermembranen zurückgehalten werden,
Überleiten von Flüssigkeit, insbesondere Wasser, aus dem Filtratraum (F_{1F}) des ersten Hohlfasermembranfilters (F1) in den Retentatraum (F_{2R}) des zweiten Hohlfasermembranfilters (F2),
Filtrieren der Flüssigkeit, insbesondere Wassers, im Retentatraum (F_{2R}) des zweiten Hohlfasermembranfilters (F2) über die Membranwandung (F_{2M}) der Hohlfasermembranen des zweiten Hohlfasermembranfilters (F2), wobei bei der Filtration Kontaminanten der Flüssigkeit von der Membranwandung (F_{2M}) der Hohlfasermembranen des zweiten Hohlfasermembranfilters (F2) zurückgehalten werden,
Ableiten von Flüssigkeit, insbesondere Wasser, aus dem Filtratraum (F_{2F}) des zweiten Hohlfasermembranfilters (F2),
gegebenenfalls Entnahme von Flüssigkeit, insbesondere Wasser oder Proben an der Entnahmestelle (E), die mit der Fluidleitung (131) zur Ableitung von Flüssigkeit, insbesondere Wasser aus dem Filtratraum (F_{2F}) des zweiten Hohlfasermembranfilters (F2) verbunden ist, und gegebenenfalls Analysieren der Proben auf Kontamination,
anschließendes Einstellen der ersten Ventilschaltung (V1) in eine durchleitende Stellung,
Einstellen der dritten Ventilschaltung (V3) in eine durchleitende Ventilstellung,
Einstellen der vierten Ventilschaltung (V4) in eine blockierende Ventilstellung,
Einstellen der ersten Ventilstellung (V1) in eine durchleitende Stellung,
nachdem eine vorherbestimmte Menge an Flüssigkeit, insbesondere Wasser, durch Filtration in den Filtratraum (F_{1F}) überführt wurde
Filtrieren der Flüssigkeit, insbesondere Wasser im Filtratraum (F_{1F}) des ersten Hohlfasermembranfilters (F1) über die Membranwandung (F_{1M}) der Hohlfasermembranen des ersten Hohlfasermembranfilters (F1) in den Retentatraum (F_{1R}) des ersten Hohlfasermembranfilters (F1), wobei bei der Filtration Kontaminanten aus dem Retentatraum (F_{1R}) des ersten Hohlfasermembranfilters (F1) freigespült werden,
Ableiten von Flüssigkeit aus dem Retentatraum (F_{1R}) des ersten Hohlfasermembranfilters (F1),
**dadurch gekennzeichnet, dass** an der ersten Probenentnahmestelle (P1) stromabwärts der ersten Ventilschaltung (V1) Proben entnommen werden und die Proben auf Kontamination analysiert werden.

11. Verfahren zur Überwachung der Aufbereitung von Flüssigkeit, insbesondere Wasser für die Dialyse, aufweisend die Schritte,
Bereitstellen einer Vorrichtung (100) nach Anspruch 3
Einstellen der ersten Ventilschaltung (V1) in eine blockierende Ventilstellung,
Einstellen der fünften Ventilschaltung (V5) in eine durchleitende Ventilstellung
Einstellen der dritten Ventilschaltung (V3) in eine blockierende Ventilstellung,
Einstellen der vierten Ventilschaltung (V4) in eine durchleitende Ventilstellung,
Einleiten von Flüssigkeit, insbesondere Wasser, in den Retentatraum (F_{1R}) des ersten Hohlfasermembranfilters (F1),
Filtrieren der Flüssigkeit, insbesondere Wasser, über die Membranwandung (F_{1M}) der Hohlfasermembranen des ersten Hohlfasermembranfilters (F1), wobei bei der Filtration Kontaminanten der Flüssigkeit, insbesondere Wasser, von der Membranwandung der Hohlfasermembranen zurückgehalten werden,
Überleiten von Flüssigkeit, insbesondere Wasser, aus dem Filtratraum (F_{1F}) des ersten Hohlfasermembranfilters (F1) in den Retentatraum (F_{2R}) des zweiten Hohlfasermembranfilters (F2),
Filtrieren der Flüssigkeit, insbesondere Wassers, im Retentatraum (F_{2R})des zweiten Hohlfasermembranfilters (F2) über die Membranwandung (F_{2M}) der Hohlfasermembranen des zweiten Hohlfasermembranfilters (F2), wobei bei der Filtration Kontaminanten der Flüssigkeit von der Membranwandung (F_{2M}) der Hohlfasermembranen des zweiten Hohlfasermembranfilters (F2) zurückgehalten werden,
Ableiten von Flüssigkeit, insbesondere Wasser, aus dem Filtratraum (F_{2F}) des zweiten Hohlfasermembranfilters (F2),
gegebenenfalls Entnahme von Flüssigkeit, insbesondere Wasser oder Proben an der Entnahmestelle (E), die mit der Fluidleitung (131) zur Ableitung von Flüssigkeit, insbesondere Wasser aus dem Filtratraum (F_{2F}) des zweiten Hohlfasermembranfilters (F2) verbunden ist, und gegebenenfalls Analysieren der Proben auf Kontamination,
anschließendes Einstellen der ersten Ventilschaltung (V1) in eine durchleitende Stellung,
Einstellen der fünften Ventilschaltung (V5) in eine blockierende Ventilstellung
Einstellen der dritten Ventilschaltung (V3) in eine durchleitende Ventilstellung,
Einstellen der vierten Ventilschaltung (V4) in eine blockierende Ventilstellung,
nachdem eine vorherbestimmte Menge an Flüssigkeit, insbesondere Wasser, durch Filtration in den Filtratraum (F_{1F}) überführt wurde,
Filtrieren der Flüssigkeit, insbesondere Wasser im Filtratraum (F_{1F}) des ersten Hohlfasermembranfilters (F1) über die Membranwandung (F_{1M}) der Hohlfasermembranen des ersten Hohlfasermembranfilters (F1) in den Retentatraum (F_{1R}) des ersten Hohlfasermembranfilters (F1), wobei bei der Filtration Kontaminanten aus dem Retentatraum (F_{1R}) des ersten Hohlfasermembranfilters (F1) freigespült werden,
Ableiten von Flüssigkeit aus dem Retentatraum (F_{1R}) des ersten Hohlfasermembranfilters (F1),
**dadurch gekennzeichnet, dass** an der ersten Probenentnahmestelle (P1) stromabwärts der ersten Ventilschaltung (V1) Proben entnommen werden und die Proben auf Kontamination analysiert werden.

12. Verfahren zur Überwachung der Aufbereitung von Flüssigkeiten, insbesondere Wasser für die Dialyse, aufweisend die Schritte,
Bereitstellen einer Vorrichtung (100) nach Anspruch 5
Einstellen der ersten Ventilschaltung (V1) in eine blockierende Stellung,
Einstellen der sechsten Ventilschaltung in eine durchleitende Stellung
Einleiten von Flüssigkeit, insbesondere Wasser, in den Retentatraum (F_{1R}) des ersten Hohlfasermembranfilters (F1),
Filtrieren von Flüssigkeit, insbesondere Wasser, über die Membranwandung (F_{1M}) der Hohlfasermembranen vom Retentatraum (F_{1R}) in den Filtratraum (F_{1F}) ersten Hohlfasermembranfilters (F1) und Auffangen von Flüssigkeit im Filtratraum (F_{1F}), wobei bei der Filtration Kontaminanten der Flüssigkeit, insbesondere Wasser, von der Hohlfasermembran zurückgehalten werden,
Überleiten von Flüssigkeit, insbesondere Wasser, vom Filtratraum (F_{1F}) des ersten Hohlfasermembranfilters (F1) in den Retentatraum (F_{2R}) des zweiten Hohlfasermembranfilters (F2),
Einstellen der sechsten Ventilschaltung (V6) in eine blockierende Stellung, nachdem eine vorherbestimmte Menge an Flüssigkeit, insbesondere Wasser, durch Filtration in den Filtratraum (F_{1F}) des ersten Hohlfasermembranfilters (F1) überführt wurde,
weiteres Einleiten von Flüssigkeit, insbesondere Wasser, in den Retentatraum (F_{1R}) des ersten Hohlfasermembranfilters (F1), Filtrieren von Flüssigkeit, insbesondere Wasser, in den Filtratraum des ersten Hohlfasermembranfilters (F_{1F}), Überleiten von Flüssigkeit, insbesondere Wasser, vom Filtratraum (F_{1F}) des ersten Hohlfasermembranfilters (F1) in den Retentatraum (F_{2R}) des zweiten Hohlfasermembranfilters (F2),
**dadurch gekennzeichnet, dass** an der vierten Probenentnahmestelle (P4) Proben entnommen werden und die Proben auf Kontamination analysiert werden.

13. Verfahren zur Überwachung der Aufbereitung von Flüssigkeit, insbesondere Wasser für die Dialyse, aufweisend die Schritte,
Bereitstellen einer Vorrichtung (100) nach Anspruch 6
Einstellen der ersten Ventilschaltung (V1) in eine blockierende Stellung,
Einstellen der fünften Ventilschaltung (V5), in eine durchleitende Stellung,
Einstellen der dritten Ventilschaltung (V3) in eine blockierende Stellung,
Einstellen der sechsten Ventilschaltung (V6) in eine durchleitende Stellung,
Einleiten von Flüssigkeit, insbesondere Wasser, in den Retentatraum (F_{1R}) des Hohlfasermembranfilters (F1),
Filtrieren der Flüssigkeit, insbesondere Wasser über die Membranwandung der Hohlfasermembranen vom Retentatraum (F_{1R}) in den Filtratraum (F_{1F}) des ersten Hohlfasermembranfilters (F1) und Auffangen von Flüssigkeit, insbesondere Wasser im Filtratraum (F_{1F}), wobei bei der Filtration Kontaminanten der Flüssigkeit, insbesondere Wasser, von der Hohlfasermembran zurückgehalten werden,
Überleiten von Flüssigkeit, insbesondere Wasser aus dem Filtratraum (F_{1F}) des ersten Hohlfasermembranfilters in den Retentatraum (F_{2R}) des zweiten Hohlfasermembranfilters (F2),
Filtrieren von Flüssigkeit, insbesondere Wasser, über die Membranwandung (F_{2M}) der Hohlfasermembranen vom Retentatraum (F_{2R}) in den Filtratraum (F_{1F}) des zweiten Hohlfasermembranfilters (F2) und Auffangen von Flüssigkeit, insbesondere Wasser, im Filtratraum (F_{1F}) des zweiten Hohlfasermembranfilters (F2), wobei bei der Filtration Kontaminanten im Rohwasser von der Hohlfasermembran zurückgehalten werden,
Ableiten von Wasser aus dem Filtratraum (F_{1F}) des zweiten Hohlfasermembranfilters und gegebenenfalls Entnahme von Flüssigkeit, insbesondere Wasser, oder Proben an der dritten Entnahmestelle (E) die mit der Fluidleitung (131) zur Ableitung von Flüssigkeit, insbesondere Wasser aus dem Filtratraum (F_{2F}) des zweiten Hohlfasermembranfilters (F2) verbunden ist, und gegebenenfalls Analysieren der Proben auf Kontamination,
anschließendes Einstellen der fünften Ventilschaltung (V5), in eine blockierende Stellung,
Einstellen der dritten Ventilschaltung (V3) in eine durchleitende Stellung,
Einstellen der sechsten Ventilschaltung (V6) in eine blockierende Stellung,
nachdem eine vorherbestimmte Menge an Flüssigkeit, insbesondere Wasser, durch Filtration in den Filtratraum (F_{1F}) überführt wurde,
Einleiten von Flüssigkeit, insbesondere Wasser in den Filtratraum (F_{2F}) des zweiten Hohlfasermembranfilters (F2), filtrieren von Flüssigkeit, insbesondere Wassers über die Membranwandung (F_{2M}) der Hohlfasermembranen vom Filtratraum (F_{2F}) in den Retentatraum (F_{2R}) des zweiten Hohlfasermembranfilters (F2) und auffangen von Flüssigkeit, insbesondere Wasser im Retentatraum (F_{2R}) des zweiten Hohlfasermembranfilters (F2), wobei bei der Filtration Kontaminanten aus dem Retentatraum des zweiten Hohlfasermembranfilters, die von den Hohlfasermembran zurückgehalten wurden freigespült werden,
**dadurch gekennzeichnet, dass** an der vierten Probenentnahmestelle (P4) Proben entnommen werden und die Proben auf Kontamination analysiert werden.

14. Verfahren nach einem der Ansprüche 8 bis 13, wobei
an einer der Probenentnahmestellen eine Probe entnommen wird und über eine Analysevorrichtung ein Analysewert bezüglich der Konzentration von Kontaminanten ermittelt wird, wobei
weiterhin das Volumen von Flüssigkeit, insbesondere Wasser, ermittelt wird, dass vom Retentatraum in den Filtratraum des ersten Hohlfasermembranfilters überführt wird, wobei
weiterhin das Volumens an Flüssigkeit, insbesondere Wasser, mit der die Kontaminanten zu der Probenentnahmestelle gespült werden, bestimmt wird, **dadurch gekennzeichnet, dass**
über eine elektronische Auswerteeinheit, die so konfiguriert, dass sie aus dem Analysewert bezüglich der Konzentration von Kontaminanten in einer jeweiligen Probe, dem Volumen der vom Retentatraum in den Filtratraum des ersten Hohlfasermembranfilters überführten Flüssigkeit, insbesondere Wasser, und dem Volumen an Flüssigkeit, insbesondere Wasser, mit der die Kontaminanten zu der Probenentnahmestelle gespült werden, ein Umrechnungsfaktor ermittelt wird, und gegebenenfalls ein Wert für eine Kontamination der für die Vorrichtung bzw. für eine Dialysemaschine bereitgestellten Flüssigkeit, insbesondere Wasser, ermittelt wird.

15. Dialysemaschine, enthaltend eine Vorrichtung (100) zur Aufbereitung einer Flüssigkeit, insbesondere zur Aufbereitung von Wasser für die Dialyse, gemäß mindestens einem der Ansprüche 1 bis 7.

## Claims

1. An apparatus (100) for the treating of a fluid, in particular for the treating of water for dialysis,
which comprises a first hollow fiber membrane filter (F1), wherein the first hollow fiber membrane filter further comprises
a plurality of hollow fiber membranes which form a retentate chamber (F_{1R}) and a filtrate chamber (F_{1F}), wherein the retentate chamber (F_{1R}) and the filtrate chamber (F_{1F}) are separated from each other by the semipermeable membrane walls (F_{1M}) of the hollow fiber membranes,
a fluid port (101) for the supplying of fluid, in particular water, to the retentate chamber,
a fluid port (102) for the draining of fluid, in particular water, from the retentate chamber,
at least one fluid port (103a I 103b) for the draining of fluid, in particular water, from the filtrate chamber,
wherein the apparatus (100) further comprises
a fluid line (110) in fluid communication with the fluid port for supplying fluid, in particular water, to the retentate chamber (F_{1R}) of the first hollow fiber membrane filter (F1),
a fluid line (111) in fluid communication with the fluid port (102) for draining fluid, in particular water, from the retentate chamber (F_{1R}) of the first hollow fiber membrane filter (F1),
a fluid line (112) in fluid communication with the fluid port (102) for draining fluid, in particular water, from the filtrate chamber (F_{1F}) of the first hollow fiber membrane filter (F1),
a first valve connection (V1) in engagement with the fluid line (111) for draining fluid, in particular water, from the retentate chamber, wherein the valve connection has at least two valve positions, by means of which the flow of fluid through the fluid line (111) for draining fluid from the retentate chamber can be blocked or conducted,
optionally further comprising
a first fluid branching point (111a) in the fluid line (111) for draining fluid, in particular water, from the retentate chamber (F_{1R}) of the first hollow fiber membrane filter (F1) in fluid communication with a first branching fluid line (113), a second valve connection (V2) in engagement with the first branching fluid line (113), wherein the second valve connection (V2) has at least two valve positions, by means of which the flow of fluid, in particular water, through the first branching fluid line (113) can be blocked or conducted,
**characterized in that**
a sampling point (P1) is arranged downstream the first valve connection (V1) in fluid communication with the fluid line (111) for draining fluid, in particular water, from the retentate chamber (F_{1R}),
and/or that
a second sampling point (P2) is arranged downstream the second valve connection (V2) in fluid communication with the first branching fluid line (113).

2. The apparatus according to claim 1 **characterized in that** a second *fluid* branching point (110a) is arranged in the fluid line (110) for supplying fluid, in particular water, to the retentate chamber (F_{1R}) upstream of the first hollow fiber membrane filter (F1),
further **characterized in that** a third fluid branching point (112) is arranged in the fluid line (112) for draining fluid, in particular water, from the filtrate chamber (F_{1F}) of the first hollow fiber membrane filter (F1),
further **characterized in that** the second fluid branching point (110a) on the fluid line (110) for supplying fluid, in particular water, into the retentate chamber of the first hollow fiber membrane filter (F1) and the third fluid branching point (112a) on the fluid line (112) for the draining of fluid, in particular water, from the filtrate chamber (F_{1F}) of the first hollow fiber membrane filter (F1) are fluidly connected by a fluid line (114),
further **characterized in that** a third valve connection (V3) is arranged in engagement with the fluid line (114) between the second fluid branching point (110a) and the third fluid branching point (112a), wherein the third valve connection (V3) has at least two valve positions, by means of which the flow of fluid, in particular water, through the fluid line (114) between the second fluid branching point (110a) and the third fluid branching point (112a) can be blocked or conducted,
further **characterized in that** a fourth valve connection (V4) is arranged downstream the third fluid branching point (112a) in engagement with the fluid line (112) for draining fluid, in particular water, from the filtrate chamber (F_{1F}), wherein the fourth valve connection (V4) has at least two valve positions, by means of which the flow of fluid, in particular water, through the fluid line (112) for draining fluid, in particular water, from the filtrate chamber can be blocked or conducted.

3. The apparatus according to claim 2, **characterized in that** a fifth valve connection (V5) is arranged upstream of the hollow fiber membrane filter (F1) between the second fluid branching point (110a) on the fluid line (110) for supplying fluid, particularly water, to the retentate chamber (F_{1R}) of the first hollow fiber membrane filter and the first hollow fiber membrane filter (F1) in engagement with the fluid line (110) for supplying fluid, in particular water, to the retentate chamber (F_{1R}) of the first hollow fiber membrane filter (F1), wherein the fifth valve connection (V5) has at least two valve positions, by means of which the flow of fluid, in particular water, through the fluid line (110) for supplying fluid, in particular water, to the retentate chamber (F_{1R}) of the first hollow fiber membrane filter (F1) can be blocked or conducted.

4. The apparatus according to at least one of claims 1 to 3, **characterized in that** the apparatus further comprises
a second hollow fiber membrane filter (F2), wherein the second hollow fiber membrane filter further comprises
a plurality of hollow fiber membranes with a retentate chamber (F_{2R}) and a filtrate chamber (F_{1F}) separated from each other by semipermeable membrane walls (F_{2M}) of the hollow fiber membranes,
a fluid port (120) for the supply of fluid, in particular water, into the retentate chamber (F_{2R}) of the second hollow fiber membrane filter,
a fluid port (121) for the draining of fluid, in particular water, from the retentate chamber (F_{2R}) of the second hollow fiber membrane filter,
at least one fluid port (123a/123b) for the draining of fluid, in particular water, from the filtrate chamber of the second hollow fiber membrane filter,
a fluid line (124) in fluid communication with the fluid port (120) for supplying fluid, in particular water, into the retentate chamber (F_{2R}) of the second hollow fiber membrane filter,
and wherein the apparatus (100) further comprises
a fluid line (130) in fluid communication with the fluid port (121) for draining fluid, in particular water, from the retentate chamber (F_{2R}) of the second hollow fiber membrane filter,
a fluid line (131) in fluid communication with the at least one fluid port (123a/123b) for draining fluid, in particular water, from the filtrate chamber (F_{2F}) of the second hollow fiber membrane filter (F2), wherein the apparatus is further **characterized in that** the fluid line (124) in fluid communication with the fluid port (120) for supplying fluid, in particular water, into the chamber (F_{2R}) chamber of the second hollow fiber membrane filter (F2) is fluidly connected to the fluid line (112) for draining fluid, in particular water, from the filtrate chamber (F_{1F}) of the first hollow fiber membrane filter (F1).

5. The apparatus according to claim 4, **characterized in that** a fourth fluid branching point (130a) is arranged in the fluid line (130) between the fluid port (121) for draining fluid, in particular water, from the retentate chamber (F_{2R}) of the second hollow fiber membrane filter (F2) from which branches a second branching fluid line (132) fluidly connected to fourth fluid branching point (130a), further **characterized in that** a sixth valve connection (V6) in engagement with the fluid line (130) for draining fluid, in particular water, from the retentate chamber (F_{2R}) of the second hollow fiber membrane filter (F2) is arranged downstream of the fourth fluid branching point (130a),
wherein the sixth valve connection (V6) has at least two valve positions, by means of which the flow of fluid, particularly water, through the fluid line (130) for draining fluid, particularly water, from the retentate chamber (F_{2R}) of the second hollow fiber membrane filter (F2) can be blocked or conducted,
further **characterized in that** the second branching fluid line (132) is in fluid communication with a fourth sampling point (P4).

6. The apparatus according to claim 1, **characterized in that** the apparatus further comprises at least one second hollow fiber membrane filter (F2),
wherein the second hollow fiber membrane filter further comprises
a plurality of hollow fiber membranes forming a retentate chamber (F_{2R}) and a filtrate chamber (F_{2F}) which are separated from each other by a semipermeable membrane wall (F_{2M}) of the hollow fiber membranes,
a fluid port (120) for the supply of fluid, in particular water, into the retentate chamber (F_{2R}) of the second hollow fiber membrane filter,
a fluid port (130) for the draining of fluid, in particular water, from the retentate chamber (F_{2R}) of the second hollow fiber membrane filter,
at least one fluid port (123a/123b) for the draining of fluid, in particular water, from the filtrate chamber (F_{2F}) of the second hollow fiber membrane filter,
and wherein the apparatus (100) further comprises
at least one fluid line (112) for supplying fluid, in particular water, into the retentate chamber (F_{2R}) of the second hollow fiber membrane filter (F2),
at least one fluid line (131) in fluid communication with the at least one fluid port (123a/123b) for draining fluid, in particular water, from the filtrate chamber (F_{2F}) of the second hollow fiber membrane filter,
wherein the apparatus (100) is further **characterized in that** a second fluid branching point (110a) is arranged in the fluid line (110) for supplying fluid, in particular water, to the retentate chamber (F_{1R}) of the first hollow fiber membrane filter (F1) upstream of the first hollow fiber membrane filter (F1), further **characterized in that** the apparatus (100) comprises a fifth fluid branching point (131a) on the fluid line (131) for draining fluid, in particular water, from the filtrate chamber (F_{2F}) of the second hollow fiber membrane filter (F2), wherein a fourth sampling point (P4) is arranged in fluid communication via a fourth fluid branching point (130a) in the fluid line (130) for draining fluid, in particular water, from the retentate chamber (F_{2R}) of the second hollow fiber membrane filter (F2),
further **characterized by** the arrangement of a fluid line (115) in fluid communication with the second fluid branching point (110a) on the fluid line (110) for supplying fluid, in particular water, into the retentate chamber (F_{1R}) of the first hollow fiber membrane filter (F1) and the fifth fluid branching point (131a) on the fluid line (131) for draining fluid, in particular water, from the filtrate chamber (F_{2F}) of the second hollow fiber membrane filter (F2), further **characterized in that** a third valve connection (V3) is arranged in engagement with the fluid line (115) between the second fluid branching point (110a) on the fluid line (110) for supplying fluid, in particular water, into the retentate chamber (F_{1R}) of the first hollow fiber membrane filter (F1) and the fifth fluid branching point (131a) on the fluid line (131) for draining fluid, in particular water, from the filtrate chamber (F_{2F}) of the second hollow fiber membrane filter (F2), wherein the third valve connection (V3) has at least two valve positions, by means of which the flow of fluid, particularly water, through the fluid line (115) between the second fluid branching point and the fifth fluid branching point can be blocked or conducted,
further **characterized in that** the fluid port (120) for supplying fluid, in particular water, to the retentate chamber (F_{2R}) of the second hollow fiber membrane filter (F2) is in fluid communication with the at least one fluid port (103a/103b) for draining fluid, in particular water, from the filtrate chamber (F_{1F}) of the first hollow fiber membrane filter (F1) via a fluid line (112),
further **characterized in that** a fifth valve connection (V5) is arranged upstream the filter (F1) between the second fluid branching point (110a) in engagement with the fluid line (110) for the supply of fluid, in particular water, into the retentate chamber (F_{1R}) and the first hollow fiber membrane filter (F1), wherein the fifth valve connection (V5) has at least two valve positions, by means of which the flow of fluid, particularly water, in the fluid line (110) for supplying fluid, particularly water, to the retentate chamber can be blocked or conducted.

7. The apparatus according to at least one of the preceding claims, wherein
the apparatus comprises an analysis apparatus, with which an analysis value relative to the concentration of contaminants in a respective sample can be determined from a sample obtained at a sampling point, wherein
the apparatus further comprises means for determining the volume of fluid, in particular water, led from the retentate chamber to the filtrate chamber of the first hollow fiber membrane filter, wherein
the apparatus further comprises means for determining the volume of fluid, in particular water, with which the contaminants are flushed to the sampling point, wherein the apparatus further comprises an electronic evaluation unit, **characterized in that**
the electronic evaluation unit is configured so as to determine a conversion factor from the analysis value on the concentration of contaminants in a respective sample, the volume of fluid, in particular water, led from the retentate chamber into the filtrate chamber of the first hollow fiber membrane filter, and the volume of fluid, in particular water, with which the contaminants are flushed to the sampling point, with which a contamination value of the fluid, in particular water, provided the apparatus can be determined.

8. A method for monitoring the treatment of fluids, in particular the treatment of water for dialysis, comprising the steps
providing an apparatus (100) according to claim 1,
setting the first valve connection (V1) and the potential second valve connection (V2) into a blocking position,
introducing fluid, particularly water, into the retentate chamber (F_{1R}) of the first hollow fiber membrane filter (F1),
filtering the fluid, in particular water, across the membrane wall (F_{1M}) of the hollow fiber membranes from the retentate chamber (F_{1R}) to the filtrate chamber (F_{1F}) of the first hollow fiber membrane filter (F1) and collecting fluid, in particular water, in the filtrate chamber (F_{1F}), whereby contaminants in the untreated water are retained by the hollow fiber membrane during filtration,
draining the fluid, in particular water, from the filtrate chamber (F_{1F}),
setting the first valve connection (V1) to a conducting position after a predetermined volume of fluid, in particular water, has passed into the filtrate chamber by filtration,
wherein the second valve connection, if applicable, remains in a blocking valve position,
introducing further fluid, particularly water, into the retentate chamber (F_{1R}) of the first hollow fiber membrane filter (F1),
**characterized in that** samples are taken at the first sampling point (P1) downstream of the first valve connection (V1) and the samples analyzed for contamination,
or, where applicable,
setting the first valve connection (V1) to a blocking valve position and setting the second valve connection to a conducting valve connection after a predetermined volume of fluid, in particular water, has passed into the filtrate chamber of the first hollow fiber membrane filter by filtration,
introducing further fluid, particularly water, into the retentate chamber (F_{1R}) of the first hollow fiber membrane filter (F1),
**characterized in that** samples are taken at the second sampling point (P2) downstream of the first fluid branching point (111a) and the samples analyzed for contamination.

9. The method according to claim 8, **characterized in that** the predetermined volume of fluid, in particular water, passed by filtration into the filtrate chamber (F_{1F}) of the first hollow fiber membrane filter amounts to at least 20 I or more, preferentially at least 100 I or more, further preferentially at least 200 I or more.

10. A method for monitoring the treatment of fluids, in particular the treatment of water for dialysis, comprising the steps
providing an apparatus (100) according to claim 2,
setting the first valve connection (V1) to a blocking valve position,
setting the third valve connection (V3) to a blocking valve position,
setting the fourth valve connection (V4) to a conducting valve position,
introducing fluid, in particular water, into the retentate chamber (F_{1R}) of the first hollow fiber membrane filter (F1),
filtering the fluid, in particular water, across the membrane wall (F_{1M}) of the hollow fiber membranes of the first hollow fiber membrane filter (F1), whereby contaminants in the fluid, in particular water, are retained by the membrane wall (F_{1M}) of the hollow fiber membranes during the filtration,
passing fluid, in particular water, from the filtrate chamber (F_{1F}) of the first hollow fiber membrane filter (F1) to the retentate chamber (F_{2R}) of the second hollow fiber membrane filter (F2),
filtering the fluid, in particular water, in the retentate chamber (F_{2R}) of the second hollow fiber membrane filter (F2) through the membrane wall (F_{1M}) of the hollow fiber membranes of the second hollow fiber membrane filter (F2), whereby contaminants in the fluid are retained by the membrane wall (F_{1M}) of the hollow fiber membranes of the second hollow fiber membrane filter (F2) during the filtration,
draining fluid, in particular water, out of the filtrate chamber (F_{2F}) of the second hollow fiber membrane filter (F2),
if needed, drawing fluid, in particular water, or samples at an extraction point (E) connected to the fluid line (131) for draining fluid, in particular water, from the filtrate chamber (F_{2F}) of the second hollow fiber membrane filter (F2), and if applicable analyzing the samples as to contamination,
subsequently setting the first valve connection (V1) to a conducting valve position,
setting the third valve connection (V3) to a conducting valve position,
setting the fourth valve connection (V4) to a blocking valve position,
setting the first valve connection (V1) to a conducting valve position,
after a predetermined volume of fluid, in particular water, has passed to the filtrate chamber (F_{1F}) by filtration,
filtering the fluid, in particular water, in the filtrate chamber (F_{1F}) of the first hollow fiber membrane filter (F1) through the membrane wall (F_{1M}) of the hollow fiber membranes of the first hollow fiber membrane filter (F1) into the retentate chamber (F_{1R}) of the first hollow fiber membrane filter (F1), whereby contaminants are flushed from the retentate chamber (F_{1R}) of the first hollow fiber membrane filter (F1) during the filtration,
draining fluid out of the retentate chamber (F_{1R}) of the first hollow fiber membrane filter (F1),
**characterized by** samples being taken at the first sampling point (P1) downstream of the first valve connection (V1) and the samples analyzed as to contamination.

11. A method for monitoring the treatment of fluids, in particular the treatment of water for dialysis, comprising the steps
providing an apparatus (100) according to claim 3,
setting the first valve connection (V1) to a blocking valve position,
setting the fifth valve connection (V5) to a conducting valve position,
setting the third valve connection (V3) to a blocking valve position,
setting the fourth valve connection (V4) to a conducting valve position,
introducing fluid, in particular water, into the retentate chamber (F_{1R}) of the first hollow fiber membrane filter (F1),
filtering the fluid, in particular water, through the membrane wall (F_{1M}) of the hollow fiber membranes of the first hollow fiber membrane filter (F1), whereby contaminants in the fluid, in particular water, are retained by the membrane wall of the hollow fiber membranes during the filtration,
passing fluid, in particular water, from the filtrate chamber (F_{1F}) of the first hollow fiber membrane filter (F1) to the retentate chamber (F_{2R}) of the second hollow fiber membrane filter (F2),
filtering the fluid, in particular water, in the retentate chamber (F_{2R}) of the second hollow fiber membrane filter (F2) through the membrane wall (F_{2M}) of the hollow fiber membranes of the second hollow fiber membrane filter (F2), whereby contaminants in the fluid are retained by the membrane wall (F_{2M}) of the hollow fiber membranes of the second hollow fiber membrane filter (F2) during the filtration,
draining fluid, in particular water, out of the filtrate chamber (F_{2F}) of the second hollow fiber membrane filter (F2),
if needed, drawing fluid, in particular water, or samples at the extraction point (E) connected to the fluid line (131) for draining fluid, in particular water, from the filtrate chamber (F_{2F}) of the second hollow fiber membrane filter (F2) and, if applicable, analyzing the sample as to contamination,
subsequently setting the first valve connection (V1) to a conducting valve position,
setting the fifth valve connection (V5) to a blocking valve position,
setting the third valve connection (V3) to a conducting valve position,
setting the fourth valve connection (V4) to a blocking valve position,
after a predetermined volume of fluid, in particular water, has passed to the filtrate chamber (F_{1F}) by filtration,
filtering the fluid, in particular water, in the filtrate chamber (F_{1F}) of the first hollow fiber membrane filter (F1) through the membrane wall (F_{1M}) of the hollow fiber membranes of the first hollow fiber membrane filter (F1) into the retentate chamber (F_{1R}) of the first hollow fiber membrane filter (F1), whereby contaminants are flushed from the retentate chamber (F_{1R}) of the first hollow fiber membrane filter (F1) during the filtration,
draining fluid out of the retentate chamber (F_{1R}) of the first hollow fiber membrane filter (F1)
**characterized in that** samples are taken at the first sampling point (P1) downstream of the first valve connection (V1) and the samples analyzed as to contamination.

12. A method for monitoring the treatment of fluids, in particular the treatment of water for dialysis, comprising the steps
providing an apparatus (100) according to claim 5,
setting the first valve connection (V1) to a blocking valve position,
setting the sixth valve connection to a conducting valve position,
introducing fluid, in particular water, into the retentate chamber (F_{1R}) of the first hollow fiber membrane filter (F1),
filtering fluid, in particular water, through the membrane wall (F_{1M}) of the hollow fiber membranes from the retentate chamber (F_{1R}) to the filtrate chamber (F_{1F}) of the first hollow fiber membrane filter (F1) and collecting fluid in the filtrate chamber (F_{1F}), whereby contaminants in the fluid, in particular water, are retained by the hollow fiber membrane during the filtration,
passing fluid, in particular water, from the filtrate chamber (F_{1F}) of the first hollow fiber membrane filter (F1) to the retentate chamber (F_{2R}) of the second hollow fiber membrane filter (F2),
setting the sixth valve connection (V6) to a blocking valve position after a predetermined volume of fluid, in particular water, has passed to the filtrate chamber (F_{1F}) of the first hollow fiber membrane filter (F1) by filtration,
further introducing of fluid, in particular water, into the retentate chamber (F_{1R}) of the first hollow fiber membrane filter (F1), filtering of fluid, in particular water, in the filtrate chamber of the first hollow fiber membrane filter (F1), passing of fluid, in particular water, from the filtrate chamber (F_{1F}) of the first hollow fiber membrane filter (F1) to the retentate chamber (F_{2R}) of the second hollow fiber membrane filter (F2),
**characterized in that** samples are taken at the fourth sampling point (P4) and the samples analyzed as to contamination.

13. A method for monitoring the treatment of fluids, in particular the treatment of water for dialysis, comprising the steps
providing an apparatus (100) according to claim 6,
setting the first valve connection (V1) to a blocking valve position,
setting the fifth valve connection (V5) to a conducting valve position,
setting the third valve connection (V3) to a blocking valve position,
setting the sixth valve connection (V6) to a conducting valve position,
introducing fluid, in particular water, into the retentate chamber (F_{1R}) of the hollow fiber membrane filter (F1),
filtering the fluid, in particular water, through the membrane wall of the hollow fiber membranes from the retentate chamber (F_{1R}) to the filtrate chamber (F_{1F}) of the first hollow fiber membrane filter (F1) and collecting fluid, in particular water, in the filtrate chamber (F_{1F}), whereby contaminants in the fluid, in particular water, are retained by the hollow fiber membrane during the filtration,
passing fluid, in particular water, from the filtrate chamber (F_{1F}) of the first hollow fiber membrane filter to the retentate chamber (F_{2R}) of the second hollow fiber membrane filter (F2),
filtering fluid, in particular water, through the membrane wall (F_{2M}) of the hollow fiber membranes from the retentate chamber (F_{2R}) to the filtrate chamber (F_{1F}) of the second hollow fiber membrane filter (F2) and collecting fluid, in particular water, in the filtrate chamber (F_{1F}) of the second hollow fiber membrane filter (F2), whereby contaminants in the untreated water are retained by the hollow fiber membrane during the filtration,
draining water out of the filtrate chamber (F_{1F}) of the second hollow fiber membrane filter and, if needed, drawing fluid, in particular water, or samples at the extraction point (E) connected to the fluid line (131) for draining fluid, in particular water, from the filtrate chamber (F_{2F}) of the second hollow fiber membrane filter (F2) and, if applicable, analyzing the sample as to contamination,
subsequently setting the fifth valve connection (V5) to a blocking valve position,
setting the third valve connection (V3) to a conducting valve position,
setting the sixth valve connection (V6) to a blocking valve position,
after a predetermined volume of fluid, in particular water, has passed to the filtrate chamber (F_{1F}) by filtration,
introducing fluid, in particular water, into the filtrate chamber (F_{2F}) of the second hollow fiber membrane filter (F2), filtering fluid, in particular water, through the membrane wall (F_{2M}) of the hollow fiber membranes from the filtrate chamber (F_{2F}) o the retentate chamber (F_{2R}) of the second hollow fiber membrane filter (F2) and collecting fluid, in particular water, in the retentate chamber (F_{2R}) of the second hollow fiber membrane filter (F2), whereby contaminants retained by the hollow fiber membrane are flushed out of the retentate chamber of the second hollow fiber membrane filter during the filtration,
**characterized in that** samples are taken at the fourth sampling point (P4) and the samples analyzed as to contamination.

14. The method according to one of claims 8 to 13, wherein
a sample is taken at one of the sampling points and an analysis value relative to the concentration of contaminants determined by means of an analysis apparatus, wherein
the volume of fluid, in particular water, passed from the retentate chamber to the filtrate chamber of the first hollow fiber membrane filter is further determined, wherein
the volume of fluid, in particular water, with which the contaminants are flushed to the sampling point is further determined,
**characterized in that**
a conversion factor is determined by means of an electronic evaluation unit configured to determine the volume of fluid, in particular water, passed from the retentate chamber to the filtrate chamber of the first hollow fiber membrane filter and the volume of fluid, in particular water, with which the contaminants are flushed to the sampling point from the analysis value on the contaminant concentration in a respective sample and, if applicable, a contamination value for the fluid, in particular water, supplied to the apparatus or dialysis machine respectively.

15. A dialysis machine comprising an apparatus (100) for the treating of a fluid, in particular for the treating of water for dialysis, according to at least one of claims 1 to 7.

## Revendications

1. Dispositif (100) pour le traitement d'un liquide, en particulier pour le traitement de l'eau pour la dialyse,
présentant un premier filtre à membrane à fibres creuses (F1), dans lequel le premier filtre à membrane à fibres creuses présente en outre
une pluralité de membranes à fibres creuses, qui forment une chambre de rétentat (F_{1R}) et une chambre de filtrat (F_{1F}), dans lequel la chambre de rétentat (F_{1R}) et la chambre de filtrat (F_{1F}) sont séparées l'une de l'autre par l'intermédiaire des parois de membranes semi-perméables (F_{1M}) des membranes à fibres creuses,
un raccord de fluide (101) pour l'amenée de liquide, en particulier de l'eau, dans la chambre de rétentat,
un raccord de fluide pour l'évacuation (102) de liquide, en particulier de l'eau, de la chambre de rétentat,
au moins un raccord de fluide (103a/ 103b) pour l'évacuation de liquide, en particulier de l'eau, de la chambre de filtrat,
dans lequel le dispositif (100) présente en outre
une conduite de fluide (110) en liaison fluidique avec le raccord de fluide pour l'amenée de liquide, en particulier de l'eau, dans la chambre de rétentat (F_{1R}) du premier filtre à membrane à fibres creuses (F1),
une conduite de fluide (111) en liaison fluidique avec le raccord de fluide (102) pour l'évacuation de liquide, en particulier de l'eau, de la chambre de rétentat (F_{1R}) du premier filtre à membrane à fibres creuses (F1),
une conduite de fluide (112) en liaison fluidique avec le raccord de fluide (102) pour l'évacuation de liquide, en particulier de l'eau, de la chambre de filtrat (F_{1F}) du premier filtre à membrane à fibres creuses (F1),
un premier circuit de soupape (V1) en prise avec la conduite de fluide (111) pour l'évacuation de liquide, en particulier de l'eau, de la chambre de rétentat, dans lequel le circuit de soupape comprend au moins deux positions de soupape, selon lesquelles l'écoulement de liquide à travers la conduite de fluide (111) pour l'évacuation de liquide de la chambre de rétentat peut être bloqué ou peut être autorisé à passer,
présentant en outre éventuellement
une première ramification de fluide (111a) dans la conduite de fluide (111) pour l'évacuation de liquide, en particulier de l'eau, de la chambre de rétentat (F_{1R}) du premier filtre à membrane à fibres creuses (F1), en liaison fluidique avec une première conduite de fluide en dérivation (113), un deuxième circuit de soupape (V2) en prise avec la première conduite de fluide en dérivation (113), dans lequel le deuxième circuit de soupape (V2) comprend au moins deux positions de soupape, selon lesquelles l'écoulement de liquide, en particulier de l'eau à travers la première conduite de fluide en dérivation (113) peut être bloqué ou peut être autorisé à passer,
**caractérisé en ce que**
un point de prélèvement d'échantillon (P1) est disposé en aval du premier circuit de soupape (V1) en liaison fluidique avec la conduite de fluide (111) pour l'évacuation de liquide, en particulier de l'eau, de la chambre de rétentat (F_{1R}),
et/ou, que
un deuxième point de prélèvement d'échantillon (P2) est disposé en aval du deuxième circuit de soupape (V2) en liaison fluidique par rapport à la première conduite de fluide en dérivation (113).

2. Dispositif selon la revendication 1, **caractérisé en ce qu'**une deuxième dérivation de fluide (110a) est disposée dans la conduite de fluide (110) pour l'amenée de liquide, en particulier de l'eau, dans la chambre de rétentat (F_{1R}) en amont du premier filtre à membrane à fibres creuses (F1),
en outre **caractérisé en ce qu'**une troisième dérivation de fluide (112a) est disposée dans la conduite de fluide (112) pour l'évacuation de liquide, en particulier de l'eau, de la chambre de filtrat (F_{1F}) du premier filtre à membrane à fibres creuses (F1),
en outre **caractérisé en ce que** la deuxième dérivation de fluide (110a) sur la conduite de fluide (110) pour l'amenée de liquide, en particulier de l'eau, dans la chambre de rétentat (F_{1F}) du premier filtre à membrane à fibres creuses (F1) et la troisième dérivation de fluide (112a) sur la conduite de fluide (112) pour l'évacuation de liquide, en particulier de l'eau, de la chambre de filtrat (F_{1F}) du premier filtre à membrane à fibres creuses (F1), sont reliées par liaison fluidique par l'intermédiaire d'une conduite de fluide (114),
en outre **caractérisé en ce qu'**un troisième circuit de soupape (V3) est disposé en prise avec la conduite de fluide (114) entre la deuxième dérivation de fluide (110a) et la troisième dérivation de fluide (112a), dans lequel le troisième circuit de soupape (V3) comprend au moins deux positions de soupape, selon lesquelles l'écoulement de liquide, en particulier de l'eau, à travers la conduite de fluide (114) entre la deuxième dérivation de fluide (110a) et la troisième dérivation de fluide (112a) peut être bloqué ou peut être autorisé à passer,
en outre **caractérisé en ce qu'**un quatrième circuit de soupape (V4) est disposé en aval de la troisième dérivation de fluide (112a) en prise avec la conduite de fluide (112) pour l'évacuation de liquide, en particulier de l'eau, de la chambre de filtrat (F_{1F}), dans lequel le quatrième circuit de soupape (V4) comprend au moins deux positions de soupape, selon lesquelles l'écoulement de liquide, en particulier de l'eau, à travers la conduite de fluide (112) pour l'évacuation de liquide, en particulier de l'eau, de la chambre de filtrat peut être bloqué ou peut être autorisé à passer.

3. Dispositif selon la revendication 2, **caractérisé en ce qu'**un cinquième circuit de soupape (V5) est disposé en amont du filtre à membrane à fibres creuses (F1) entre la deuxième dérivation de fluide (110a) sur la conduite de fluide (110) pour l'amenée de liquide, en particulier de l'eau dans la chambre de rétentat (F_{1R}) du premier filtre à membrane à fibres creuses et le filtre à membrane à fibres creuses (F1) en prise avec la conduite de fluide (110) pour l'amenée de liquide, en particulier de l'eau dans la chambre de rétentat (F_{1R}) du premier filtre à membrane à fibres creuses (F1), dans lequel le cinquième circuit de soupape (V5) comprend au moins deux positions de soupape, selon lesquelles l'écoulement de liquide, en particulier de l'eau, à travers la conduite de fluide (110) pour l'amenée de liquide, en particulier de l'eau dans la chambre de rétentat (F_{1R}) du premier filtre à membrane à fibres creuses (F1), peut être bloqué ou peut être autorisé à passer.

4. Dispositif selon au moins l'une des revendications 1 à 3, **caractérisé en ce que** le dispositif présente en outre
un deuxième filtre à membrane à fibres creuses (F2), dans lequel le deuxième filtre à membrane à fibres creuses présente en outre
une pluralité de membranes à fibres creuses, avec une chambre de rétentat (F_{2R}) et une chambre de filtrat (F_{1F}), qui sont séparées l'une de l'autre par l'intermédiaire de parois de membranes semi-perméables (F_{2M}) des membranes à fibres creuses,
un raccord de fluide (120) pour l'amenée de liquide, en particulier de l'eau dans la chambre de rétentat (F_{2R}) du deuxième filtre à membrane à fibres creuses,
un raccord de fluide (121) pour l'évacuation de liquide, en particulier de l'eau de la chambre de rétentat (F_{2R}) du deuxième filtre à membrane à fibres creuses,
au moins un raccord de fluide (123a/ 123b) pour l'évacuation de liquide, en particulier de l'eau de la chambre de filtrat du deuxième filtre à membrane à fibres creuses,
une conduite de fluide (124) en liaison fluidique avec le raccord de fluide (120) pour l'amenée de liquide, en particulier de l'eau dans la chambre de rétentat (F_{2R}) du deuxième filtre à membrane à fibres creuses,
et dans lequel le dispositif (100) présente en outre
une conduite de fluide (130) en liaison fluidique avec le raccord de fluide (121) pour l'évacuation de liquide, en particulier de l'eau de la chambre de rétentat (F_{2R}) du deuxième filtre à membrane à fibres creuses,
une conduite de fluide (131) en liaison fluidique avec le au moins un raccord de fluide (123a/ 123b) pour l'évacuation de liquide, en particulier de l'eau, de la chambre de filtrat (F_{2F}) du deuxième filtre à membrane à fibres creuses (F2), dans lequel le dispositif est en outre **caractérisé en ce que** la conduite de fluide (124) en liaison fluidique avec le raccord de fluide (120) pour l'amenée de liquide, en particulier de l'eau, dans la chambre de rétentat (F_{2R}) du deuxième filtre à membrane à fibres creuses (F2) est reliée par liaison fluidique à la conduite de fluide (112) pour l'évacuation de liquide, en particulier de l'eau, de la chambre de filtrat (F_{1F}) du premier filtre à membrane à fibres creuses (F1).

5. Dispositif selon la revendication 4, **caractérisé en ce qu'**une quatrième dérivation de fluide (130a), de laquelle dérive une deuxième conduite de fluide en dérivation (132) en liaison fluidique par rapport à la ramification de fluide (130a), est disposée dans la conduite de fluide (130) entre le raccord de fluide (121) pour l'évacuation de liquide, en particulier de l'eau, de la chambre de rétentat (F_{2R}) du deuxième filtre à membrane à fibres creuses (F2), en outre **caractérisé en ce qu'**un sixième circuit de soupape (V6) en prise avec la conduite de fluide (130) pour l'évacuation de liquide, en particulier de l'eau, de la chambre de rétentat (F_{2F}) du deuxième filtre à membrane à fibres creuses (F2) est disposé en aval de la quatrième dérivation de fluide (130a), dans lequel le sixième circuit de soupape (V6) comprend au moins deux positions de soupape, selon lesquelles l'écoulement de liquide, en particulier de l'eau, à travers la conduite de fluide (130) pour l'évacuation de liquide, en particulier de l'eau, de la chambre de rétentat (F_{2R}) du deuxième filtre à membrane à fibres creuses (F2), peut être bloqué ou peut être autorisé à passer ;
en outre **caractérisé en ce que** la deuxième conduite de fluide en dérivation (132) est en liaison fluidique avec un quatrième point de prélèvement d'échantillon (P4).

6. Dispositif selon la revendication 1, **caractérisé en ce que** le dispositif présente en outre au moins un deuxième filtre à membrane à fibres creuses (F2),
dans lequel le deuxième filtre à membrane à fibres creuses présente
une pluralité de membranes à fibres creuses, qui forment une chambre de rétentat (F_{2R}) et une chambre de filtrat (F_{2F}), qui sont séparées l'une de l'autre par l'intermédiaire d'une paroi de membrane semi-perméable (F_{2M}) des membranes à fibres creuses,
un raccord de fluide (120) pour l'amenée de liquide, en particulier de l'eau, dans la chambre de rétentat (F_{2R}) du deuxième filtre à membrane à fibres creuses,
un raccord de fluide (121) pour l'évacuation de liquide, en particulier de l'eau, de la chambre de rétentat (F_{2R}) du deuxième filtre à membrane à fibres creuses,
au moins un raccord de fluide (123a/ 123b) pour l'évacuation de liquide, en particulier de l'eau, de la chambre de filtrat (F_{2F}) du deuxième filtre à membrane à fibres creuses,
et dans lequel le dispositif (100) présente en outre
au moins une conduite de fluide (112) pour l'amenée de liquide, en particulier de l'eau dans la chambre de rétentat (F_{2F}) du deuxième filtre à membrane à fibres creuses (F2),
au moins une conduite de fluide (131) en liaison fluidique avec le au moins un raccord de fluide (123a/ 123b) pour l'évacuation de liquide, en particulier de l'eau, de la chambre de filtrat (F_{2F}) du deuxième filtre à membrane à fibres creuses,
dans lequel le dispositif (100) est en outre **caractérisé en ce qu'**une deuxième ramification de fluide (110a) est disposée dans la conduite de fluide (110) pour l'amenée de liquide, en particulier de l'eau, dans la chambre de rétentat (F_{1R}) du premier filtre à membrane à fibres creuses (F1) en amont du premier filtre à membrane à fibres creuses (F1), en outre **caractérisé en ce que** le dispositif (100) sur la conduite de fluide (131) pour l'évacuation de liquide, en particulier de l'eau, de la chambre de filtrat (F_{2F}) du deuxième filtre à membrane à fibres creuses (F2) présente une cinquième ramification de fluide (131a), dans lequel un quatrième point de prélèvement d'échantillon (P4) est disposé en liaison fluidique par l'intermédiaire d'un quatrième point de ramification (130a) dans la conduite de fluide (130) pour l'évacuation de liquide, en particulier de l'eau, de la chambre de rétentat (F_{2R}) du deuxième filtre à membrane à fibres creuses (F2),
en outre **caractérisé en ce qu'**une conduite de fluide (115) en liaison fluidique avec la deuxième ramification de fluide (110a) est disposée sur la conduite de fluide (110) pour l'amenée de liquide, en particulier de l'eau, dans la chambre de rétentat (F_{1R}) du premier filtre à membrane à fibres creuses (F1) et la cinquième ramification de fluide (131a) sur la conduite de fluide (131) pour l'évacuation de liquide, en particulier de l'eau, de la chambre de filtrat (F_{2F}) du deuxième filtre à membrane à fibres creuses (F2), en outre **caractérisé en ce qu'**un troisième circuit de soupape (V3) est disposé en prise avec la conduite de fluide (115) entre la deuxième ramification de fluide (110a) sur la conduite de fluide (110) pour l'amenée de liquides, en particulier de l'eau, dans la chambre de rétentat (F_{1R}) premier filtre à membrane à fibres creuses (F1) et la cinquième ramification de fluide (131a) sur la conduite de fluide (131) pour l'évacuation de liquide, en particulier de l'eau de la chambre de filtrat (F_{2F}) du deuxième filtre à membrane à fibres creuses (F2), dans lequel le troisième circuit de soupape (V3) comprend au moins deux positions de soupape, selon lesquelles l'écoulement de liquide, en particulier de l'eau, à travers la conduite de fluide (115) entre la deuxième dérivation de fluide et la cinquième dérivation de fluide peut être bloqué ou peut être autorisé à passer,
en outre **caractérisé en ce que** le raccord de fluide (120) pour l'amenée de liquide, en particulier de l'eau, dans la chambre de rétentat (F_{2R}) du deuxième filtre à membrane à fibres creuses (F2) est en liaison fluidique avec le au moins un raccord de fluide (103a/ 103b) pour l'évacuation de liquide, en particulier de l'eau, de la chambre de filtrat (F_{1F}) du premier filtre à membrane à fibres creuses (F1) par l'intermédiaire d'une conduite de fluide (112),
en outre **caractérisé en ce qu'**un cinquième circuit de soupape (V5) est disposé en amont du filtre (F1) entre la deuxième ramification de fluide (110a) en prise avec la conduite de fluide (110) pour l'amenée de liquide, en particulier de l'eau, dans la chambre de rétentat (F_{1R}) et le premier filtre à membrane à fibres creuses (F1), dans lequel le cinquième circuit de soupape (V5) comprend au moins deux positions de soupape, selon lesquelles l'écoulement de liquide, en particulier de l'eau, à travers la conduite de fluide (110) pour l'amenée de liquide, en particulier de l'eau, dans la chambre de rétentat peut être bloqué ou peut être autorisé à passer.

7. Dispositif selon au moins l'une des revendications précédentes, dans lequel
le dispositif présente un dispositif d'analyse, avec lequel une valeur d'analyse concernant la concentration de contaminants dans un échantillon respectif sur un point de prélèvement d'échantillon d'un échantillon obtenu peut être établie, dans lequel
le dispositif présente en outre des moyens pour la détermination du volume du liquide, en particulier de l'eau, transporté à partir de la chambre de rétentat dans la chambre de filtrat du premier filtre à membrane à fibres creuses, dans lequel
le dispositif présente en outre des moyens pour la détermination du volume de liquide, en particulier de l'eau, avec lequel les contaminants sont balayés vers le point de prélèvement d'échantillon, dans lequel le dispositif présente en outre une unité d'évaluation électronique, **caractérisé en ce que**
l'unité évaluation est configurée de sorte qu'elle établit à partir de la valeur d'analyse concernant la concentration de contaminants dans un échantillon respectif, le volume du liquide transporté à partir de chambre de rétentat dans la chambre de filtrat du premier filtre à membrane à fibres creuses, en particulier de l'eau, et le volume de liquide, en particulier de l'eau, avec lequel les contaminants sont balayés vers le point de prélèvement d'échantillon, établit un facteur de conversion, avec lequel une valeur pour une contamination du liquide fourni pour le dispositif, en particulier de l'eau, est établie.

8. Procédé pour la surveillance du traitement de liquides, en particulier pour le traitement de l'eau pour la dialyse, présentant les étapes de
fourniture d'un dispositif (100) selon la revendication 1
réglage du premier circuit de soupape (V1) et du éventuellement deuxième circuit de soupape (V2) dans une position de blocage,
introduction de liquide, en particulier de l'eau, dans la chambre de rétentat (F_{1R}) du premier filtre à membrane à fibres creuses (F1),
filtration du liquide, en particulier de l'eau par l'intermédiaire de la paroi de membrane (F_{1M}) des membranes à fibres creuses à partir de la chambre de rétentat (F_{1R}) dans la chambre de filtrat (F_{1F}) du premier filtre à membrane à fibres creuses (F1) et collecte du liquide, en particulier de l'eau, dans la chambre de filtrat (F_{1F}), dans lequel lors de la filtration les contaminants dans l'eau brute sont retenus par la membrane à fibres creuses,
évacuation du liquide, en particulier de l'eau, de la chambre de filtrat (F_{1F}),
réglage du premier circuit de soupape (V1) dans une position de passage, après qu'une quantité prédéterminée de liquide, en particulier de l'eau, a été transportée par filtration dans la chambre de filtrat,
dans lequel le éventuellement deuxième circuit de soupape reste dans une position de soupape de blocage,
poursuite d'introduction de liquide, en particulier de l'eau, dans la chambre de rétentat (F_{1R}) du premier filtre à membrane à fibres creuses (F1),
**caractérisé en ce que** des échantillons sont prélevés sur le premier point de prélèvement d'échantillon (P1) en aval du premier circuit de soupape (V1) et les échantillons sont analysés à la recherche d'une contamination,
ou éventuellement
réglage du premier circuit de soupape (V1) dans une position de soupape de blocage, et
réglage du deuxième circuit de soupape dans une position de soupape de passage après qu'une quantité prédéterminée de liquide, en particulier de l'eau, a été transportée par filtration dans la chambre de filtrat,
poursuite d'introduction de liquide, en particulier de l'eau, dans la chambre de rétentat (F_{1R}) du premier filtre à membrane à fibres creuses (F1),
**caractérisé en ce que** des échantillons sont prélevés sur le deuxième point de prélèvement d'échantillon (P2) en aval de la première ramification de fluide (111a) et les échantillons sont analysés à la recherche d'une contamination.

9. Procédé selon la revendication 8, **caractérisé en ce que** la quantité prédéterminée de liquide, en particulier de l'eau, qui a été transportée par filtration dans la chambre de filtrat (F_{1F}) du premier filtre à membrane à fibres creuses, atteint au moins 20 l, ou plus, de préférence au moins 100 1 ou plus, plus préférablement au moins 200 1 ou plus.

10. Procédé pour la surveillance du traitement de liquides, en particulier de l'eau pour la dialyse, présentant les étapes de
fourniture d'un dispositif (100) selon la revendication 2 réglage du premier circuit de soupape (V1) dans une position de soupape de blocage,
réglage du troisième circuit de soupape (V3) dans une position de soupape de blocage,
réglage du quatrième circuit de soupape (V4) dans une position de soupape de passage,
introduction de liquide, en particulier de l'eau, dans la chambre de rétentat (F_{1R}) du premier filtre à membrane à fibres creuses (F1),
filtration du liquide, en particulier de l'eau, par l'intermédiaire de la paroi de membrane (F_{1M}) des membranes à fibres creuses du premier filtre à membrane à fibres creuses (F1), dans lequel lors de la filtration les contaminants du liquide, en particulier de l'eau, sont retenus par la paroi de membrane (F_{1M}) des membranes à fibres creuses,
transfert de liquide, en particulier de l'eau, à partir de la chambre de filtrat (F_{1F}) du premier filtre à membrane à fibres creuses (F1) dans la chambre de rétentat (F_{2R}) du deuxième filtre à membrane à fibres creuses (F2),
filtration du liquide, en particulier de l'eau, dans la chambre de rétentat (F_{2R}) du deuxième filtre à membrane à fibres creuses (F2) par l'intermédiaire de la paroi de membrane (F_{2M}) des membranes à fibres creuses du deuxième filtre à membrane à fibres creuses (F2), dans lequel lors de la filtration les contaminants du liquide sont retenus par la paroi de membrane (F_{2M}) des membranes à fibres creuses du deuxième filtre à membrane à fibres creuses (F2),
évacuation de liquide, en particulier de l'eau, de la chambre de filtrat (F_{2F}) du deuxième filtre à membrane à fibres creuses (F2),
éventuellement prélèvement de liquide, en particulier de l'eau ou d'échantillons sur le point de prélèvement (E), qui est relié à la conduite de fluide (131) pour l'évacuation de liquide, en particulier de l'eau de la chambre de filtrat (F_{2F}) du deuxième filtre à membrane à fibres creuses (F2), et éventuellement analyse des échantillons à la recherche d'une contamination,
ensuite réglage du premier circuit de soupape (V1) dans une position de passage,
réglage du troisième circuit de soupape (V3) dans une position de soupape de passage,
réglage du quatrième circuit de soupape (V4) dans une position de soupape de blocage,
réglage de la première position de soupape (V1) dans une position de passage,
après qu'une quantité prédéterminée de liquide, en particulier de l'eau, a été transportée par filtration dans la chambre de filtrat (F_{1F})
filtration du liquide, en particulier de l'eau dans la chambre de filtrat (F_{1F}) du premier filtre à membrane à fibres creuses (F1) par l'intermédiaire de la paroi de membrane (F_{1M}) des membranes à fibres creuses du premier filtre à membrane à fibres creuses (F1) dans la chambre de rétentat (F_{1R}) du premier filtre à membrane à fibres creuses (F1), dans lequel lors de la filtration les contaminants sont libérés de la chambre de rétentat (F_{1R}) du premier filtre à membrane à fibres creuses (F1) par rinçage,
évacuation de liquide de la chambre de rétentat (F_{1R}) du premier filtre à membrane à fibres creuses (F1),
**caractérisé en ce que** des échantillons sont prélevés sur le premier point de prélèvement d'échantillon (P1) en aval du premier circuit de soupape (V1) et les échantillons sont analysés à la recherche d'une contamination.

11. Procédé pour la surveillance du traitement de liquide, en particulier de l'eau pour la dialyse, présentant les étapes de
fourniture d'un dispositif (100) selon la revendication 3
réglage du premier circuit de soupape (V1) dans une position de soupape de blocage,
réglage du cinquième circuit de soupape (V5) dans une position de soupape de passage
réglage du troisième circuit de soupape (V3) dans une position de soupape de blocage,
réglage du quatrième circuit de soupape (V4) dans une position de soupape de passage,
introduction de liquide, en particulier de l'eau, dans la chambre de rétentat (F_{1R}) du premier filtre à membrane à fibres creuses (F1),
filtration du liquide, en particulier de l'eau, par l'intermédiaire de la paroi de membrane (F_{1M}) des membranes à fibres creuses du premier filtre à membrane à fibres creuses (F1), dans lequel lors de la filtration les contaminants du liquide, en particulier de l'eau, sont retenus par la paroi de membrane des membranes à fibres creuses,
transfert de liquide, en particulier de l'eau, à partir de la chambre de filtrat (F_{1F}) du premier filtre à membrane à fibres creuses (F1) dans la chambre de rétentat (F_{2R}) du deuxième filtre à membrane à fibres creuses (F2),
filtration du liquide, en particulier de l'eau, dans la chambre de rétentat (F_{2R}) du deuxième filtre à membrane à fibres creuses (F2) par l'intermédiaire de la paroi de membrane (F_{2M}) des membranes à fibres creuses du deuxième filtre à membrane à fibres creuses (F2), dans lequel lors de la filtration les contaminants du liquide sont retenus par la paroi de membrane (F_{2M}) des membranes à fibres creuses du deuxième filtre à membrane à fibres creuses (F2),
évacuation de liquide, en particulier de l'eau, de la chambre de filtrat (F_{2F}) du deuxième filtre à membrane à fibres creuses (F2),
éventuellement prélèvement de liquide, en particulier de l'eau ou d'échantillons sur le point de prélèvement (E), qui est relié à la conduite de fluide (131) pour l'évacuation de liquide, en particulier de l'eau de la chambre de filtrat (F_{2F}) du deuxième filtre à membrane à fibres creuses (F2), et éventuellement analyse des échantillons à la recherche d'une contamination,
ensuite réglage du premier circuit de soupape (V1) dans une position de passage,
réglage du cinquième circuit de soupape (V5) dans une position de soupape de blocage
réglage du troisième circuit de soupape (V3) dans une position de soupape de passage,
réglage du quatrième circuit de soupape (V4) dans une position de soupape de blocage,
après qu'une quantité prédéterminée de liquide, en particulier de l'eau, a été transportée par filtration dans la chambre de filtrat (F_{1F}),
filtration du liquide, en particulier de l'eau dans la chambre de filtrat (F_{1F}) du premier filtre à membrane à fibres creuses (F1) par l'intermédiaire de la paroi de membrane (F_{1M}) des membranes à fibres creuses du premier filtre à membrane à fibres creuses (F1) dans la chambre de rétentat (F_{1R}) du premier filtre à membrane à fibres creuses (F1), dans lequel lors de la filtration les contaminants sont libérés de la chambre de rétentat (F_{1R}) du premier filtre à membrane à fibres creuses (F1) par rinçage,
évacuation de liquide de la chambre de rétentat (F_{1R}) du premier filtre à membrane à fibres creuses (F1),
**caractérisé en ce que** des échantillons sont prélevés sur le premier point de prélèvement d'échantillon (P1) en aval du premier circuit de soupape (V1) et les échantillons sont analysés à la recherche d'une contamination.

12. Procédé pour la surveillance du traitement de liquides, en particulier de l'eau pour la dialyse, présentant les étapes de
fourniture d'un dispositif (100) selon la revendication 5
réglage du premier circuit de soupape (V1) dans une position de blocage,
réglage du sixième circuit de soupape dans une position de passage
introduction de liquide, en particulier de l'eau, dans la chambre de rétentat (F_{1R}) du premier filtre à membrane à fibres creuses (F1),
filtration de liquide, en particulier de l'eau, par l'intermédiaire de la paroi de membrane (F_{1M}) des membranes à fibres creuses à partir de la chambre de rétentat (F_{1R}) dans la chambre de filtrat (F_{1F}) du premier filtre à membrane à fibres creuses (F1) et collecte de liquide dans la chambre de filtrat (F_{1F}), dans lequel lors de la filtration les contaminants du liquide, en particulier de l'eau, sont retenus par la membrane à fibres creuses,
transfert de liquide, en particulier de l'eau, à partir de la chambre de filtrat (F_{1F}) du premier filtre à membrane à fibres creuses (F1) dans la chambre de rétentat (F_{2R}) du deuxième filtre à membrane à fibres creuses (F2),
réglage du sixième circuit de soupape (V6) dans une position de blocage, après qu'une quantité prédéterminée de liquide, en particulier de l'eau, a été transportée par filtration dans la chambre de filtrat (F_{1F}) du premier filtre à membrane à fibres creuses (F1),
poursuite d'introduction de liquide, en particulier de l'eau, dans la chambre de rétentat (F_{1R}) du premier filtre à membrane à fibres creuses (F1), filtration de liquide, en particulier de l'eau, dans la chambre de filtrat du premier filtre à membrane à fibres creuses (F_{1F}), transfert de liquide, en particulier de l'eau, à partir de la chambre de filtrat (F_{1F}) du premier filtre à membrane à fibres creuses (F1) dans la chambre de rétentat (F_{2R}) du deuxième filtre à membrane à fibres creuses (F2),
**caractérisé en ce que** des échantillons sont prélevés sur le quatrième point de prélèvement d'échantillon (P4) et les échantillons sont analysés à la recherche d'une contamination.

13. Procédé pour la surveillance du traitement de liquide, en particulier de l'eau pour la dialyse, présentant les étapes de
fourniture d'un dispositif (100) selon la revendication 6
réglage du premier circuit de soupape (V1) dans une position de blocage,
réglage du cinquième circuit de soupape (V5) dans une position de passage,
réglage du troisième circuit de soupape (V3) dans une position de blocage,
réglage du sixième circuit de soupape (V6) dans une position de passage,
introduction de liquide, en particulier de l'eau, dans la chambre de rétentat (F_{1R}) du filtre à membrane à fibres creuses (F1),
filtration du liquide, en particulier de l'eau, par l'intermédiaire de la paroi de membrane des membranes à fibres creuses à partir de la chambre de rétentat (F_{1R}) dans la chambre de filtrat (F_{1F}) du premier filtre à membrane à fibres creuses (F1) et collecte de liquide, en particulier de l'eau, dans la chambre de filtrat (F_{1F}), dans lequel lors de la filtration les contaminants du liquide, en particulier de l'eau, sont retenus par la membrane à fibres creuses,
transfert de liquide, en particulier de l'eau à partir de la chambre de filtrat (F_{1F}) du premier filtre à membrane à fibres creuses dans la chambre de rétentat (F_{2R}) du deuxième filtre à membrane à fibres creuses (F2),
filtration de liquide, en particulier de l'eau, par l'intermédiaire de la paroi de membrane (F_{2M}) des membranes à fibres creuses à partir de la chambre de rétentat (F_{2R}) dans la chambre de filtrat (F_{1F}) du deuxième filtre à membrane à fibres creuses (F2) et collecte de liquide, en particulier de l'eau, dans la chambre de filtrat (F_{1F}) du deuxième filtre à membrane à fibres creuses (F2), dans lequel lors de la filtration les contaminants dans l'eau brute sont retenus par la membrane à fibres creuses,
évacuation de l'eau de la chambre de filtrat (F_{1F}) du deuxième filtre à membrane à fibres creuses et éventuellement prélèvement de liquide, en particulier de l'eau, ou d'échantillons sur le troisième point de prélèvement (E) qui est relié à la conduite de fluide (131) pour l'évacuation de liquide, en particulier de l'eau de la chambre de filtrat (F_{2F}) du deuxième filtre à membrane à fibres creuses (F2), et éventuellement analyse des échantillons à la recherche d'une contamination,
ensuite réglage du cinquième circuit de soupape (V5) dans une position de blocage,
réglage du troisième circuit de soupape (V3) dans une position de passage,
réglage du sixième circuit de soupape (V6) dans une position de blocage,
après qu'une quantité prédéterminée de liquide, en particulier de l'eau, a été transportée par filtration dans la chambre de filtrat (F_{1F}),
introduction de liquide, en particulier de l'eau dans la chambre de filtrat (F_{2F}) du deuxième filtre à membrane à fibres creuses (F2), filtration de liquide, en particulier de l'eau par l'intermédiaire de la paroi de membrane (F_{2M}) des membranes à fibres creuses à partir de la chambre de filtrat (F_{2F}) dans la chambre de rétentat (F_{2R}) du deuxième filtre à membrane à fibres creuses (F2) et collecte de liquide, en particulier de l'eau dans la chambre de rétentat (F_{2R}) du deuxième filtre à membrane à fibres creuses (F2), dans lequel lors de la filtration les contaminants qui ont été retenus par les membranes à fibres creuses sont libérés de la chambre de rétentat du deuxième filtre à membrane à fibres creuses par rinçage,
**caractérisé en ce que** des échantillons sont prélevés sur le quatrième point de prélèvement d'échantillon (P4) et les échantillons sont analysés à la recherche d'une contamination.

14. Procédé selon l'une quelconque des revendications 8 à 13, dans lequel
un échantillon est prélevé sur un des points de prélèvement d'échantillon et une valeur d'analyse concernant la concentration de contaminants est établie par l'intermédiaire d'un dispositif d'analyse, dans lequel
par ailleurs le volume de liquide, en particulier de l'eau, qui est transporté à partir de la chambre de rétentat dans la chambre de filtrat du premier filtre à membrane à fibres creuses, est établi, dans lequel
par ailleurs le volume de liquide, en particulier de l'eau, avec lequel les contaminants sont balayés vers le point de prélèvement d'échantillon, est déterminé **caractérisé en ce que**
par l'intermédiaire d'une unité d'évaluation électronique, qui est configurée de sorte qu'à partir de la valeur d'analyse concernant la concentration de contaminants dans un échantillon respectif, du volume du liquide, en particulier de l'eau, transporté à partir de la chambre de rétentat dans la chambre de filtrat du premier filtre à membrane à fibres creuses, et du volume de liquide, en particulier de l'eau, avec lequel les contaminants sont balayés vers le point de prélèvement d'échantillon, un facteur de conversion est établi, et éventuellement une valeur pour une contamination du liquide, en particulier de l'eau, fourni pour le dispositif ou pour une machine de dialyse, est établie.

15. Machine de dialyse, contenant un dispositif (100) pour le traitement d'un liquide, en particulier pour le traitement de l'eau pour la dialyse, selon au moins l'une des revendications 1 à 7.
